# EUROPEAN PATENT SPECIFICATION

(11) **EP 3 828 281 B1**
(45) Date of publication and mention of the grant of the patent: **27.11.2024**
(21) Application number: 20214665.0
(22) Date of filing: 27.01.2017
(51) Int. Cl.: C12Q 1/68

(54) **METHODS FOR DETECTING CPG METHYLATION OF TUMOR-DERIVED DNA IN BLOOD SAMPLES**
VERFAHREN FÜR DEN NACHWEIS VON CPG-METHYLIERUNG VON TUMORABGELEITETER DNA IN BLUTPROBEN
PROCÉDÉS DE DÉTECTION DE LA MÉTHYLATION CPG DE L'ADN DÉRIVÉ DE TUMEURS DANS DES ÉCHANTILLONS DE SANG

(30) Priority: 29.01.2016 EP 16153452
(43) Date of publication of application: 02.06.2021
(62) Divisional of application: 17702586.3
(73) Proprietor: New Day Diagnostics LLC, Knoxville, TN 37919 (US)
(72) Inventor: Lewin, Jörn, 10115 Berlin (DE); Kottwitz, Denise, 12109 Berlin (DE); Esche, Selina, 14482 Potsdam (DE)
(74) Representative: Dehns

(56) References cited:
- WO-A1-00/47600
- WO-A1-2013/177265
- WO-A2-2014/036314
- WO-A2-2015/187823
- REJANE HUGHES CARVALHO ET AL: "Genome-wide DNA methylation profiling of non-small cell lung carcinomas", EPIGENETICS & CHROMATIN, BIOMED CENTRAL LTD, LONDON, UK, vol. 5, no. 1, 22 June 2012 (2012-06-22), pages 9, XP021127600, ISSN: 1756-8935, DOI: 10.1186/1756-8935-5-9
- A. VINCENT ET AL: "Genome-Wide Analysis of Promoter Methylation Associated with Gene Expression Profile in Pancreatic Adenocarcinoma", CLINICAL CANCER RESEARCH, vol. 17, no. 13, 24 May 2011 (2011-05-24), pages 4341 - 4354, XP055092899, ISSN: 1078-0432, DOI: 10.1158/1078-0432.CCR-10-3431
- ILLUMINA: "Infinium HumanMethylation27, RevB BeadChip Kits", INTERNET CITATION, 8 February 2011 (2011-02-08), pages 1 - 2, XP002621307, Retrieved from the Internet <URL:http://www.illumina.com/products/infinium_humanmethylation27_beadchip_kits.ilmn> [retrieved on 20110208]
- HONG-PAN TIAN ET AL: "DNA Methylation Affects the SP1-regulated Transcription of FOXF2 in Breast Cancer Cells", JOURNAL OF BIOLOGICAL CHEMISTRY, vol. 290, no. 31, 12 June 2015 (2015-06-12), US, pages 19173 - 19183, XP055282490, ISSN: 0021-9258, DOI: 10.1074/jbc.M114.636126

## Description

### FIELD OF THE INVENTION

The present invention relates to the field of pharmacogenomics and in particular to detecting the presence or absence of methylated ANKRD13B and/or FOXF2 DNA derived from a tumor in blood or blood-derived samples. This detection is useful for a minimally invasive diagnosis of cancers and the invention provides methods and oligonucleotides suitable for this purpose.

### BACKGROUND OF THE INVENTION

More than 65 years ago Mandel and Metais described for the first time their observation of the presence of extracellular nucleic acids in humans (Mandel P, Metais P. Les acides nucleiques du plasma sanguin chez l'homme. C.R.Acad.Sci.Paris 142, 241-243. 1948) and more than four decades later it could be clearly demonstrated that tumor-associated genetic alterations can be found in cell-free nucleic acids isolated from plasma, serum and other body fluids (Fleischhacker M, Schmidt B. (2007) Circulating nucleic acids (CNAs) and cancer--a survey. Biochim Biophys Acta 1775: 181-232; Jung K, Fleischhacker M, Rabien A. (2010) Cell-free DNA in the blood as a solid tumor biomarker-a critical appraisal of the literature. Clin Chim Acta 411: 1611-1624). This includes epigenetic alterations observed in different forms of malignant tumors. A hallmark of mammalian chromatin is DNA methylation and it is known that cytosine methylation in the context of a CpG dinucleotide plays a role in the regulation of development and is important in basic biological processes like embryogenesis and cell differentiation (Smith ZD, Meissner A. (2013) DNA methylation: roles in mammalian development. Nat Rev Genet 14: 204-220; Gibney ER, Nolan CM. (2010) Epigenetics and gene expression. Heredity (Edinb) 105: 4-13). As such, methylation not only regulates gene transcription, but also plays a role in maintaining genome stability, imprinting and X-chromosome inactivation. Epigenetic alterations in oncogenes and tumor suppressor genes are of key importance in the development of cancer (Suva ML, Riggi N, Bernstein BE. (2013) Epigenetic reprogramming in cancer. Science 339: 1567-1570). DNA methylation patterns are largely modified in cancer cells and can therefore be used to distinguish cancer cells from normal tissues. As such, DNA methylation patterns are being used to diagnose all sorts of cancers. Genome-wide DNA methylation profiling of non-small cell lung carcinomas has been described (Carvalho RH et al. (2012) Genome-wide DNA methylation profiling of non-small cell lung carcinomas. Epigenetics & Chromatin 5:9). A relatively recent concept is the use of free circulating tumor DNA that is released from the tumor for example into the blood for methylation analysis as an indicator for tumor load in the body of the patient. This ability to isolate and to characterize extracellular nucleic acids from tumor patients with very sensitive and highly specific methods led to the term "liquid biopsy". As a result, physicians no longer solely depend on the examination of tissue biopsies and body scans.

The less advanced the cancer is, the better the treatment options and the chances of curing the patient are. Thus, it is highly desirable to diagnose a cancer as early as possible. However, a less advanced cancer, which means smaller tumor size and less cancer cells, releases less free circulating tumor DNA. This is exacerbated by the fact that the half-life of extracellular nucleic acids is rather short, for example less than six hours in plasma (Rago C, Huso DL, Diehl F, Karim B, Liu G, et al. (2007) Serial assessment of human tumor burdens in mice by the analysis of circulating DNA. Cancer Res 67: 9364-9370). A further difficulty is that small amounts of methylated tumor marker DNA have to be detected at high backgrounds of non-tumor DNA, which greatly challenges the sensitivity of the detection methods. Very good results have been achieved using technologies based on a selective amplification of methylated tumor DNA after bisulfite conversion, like methylation-specific PCR (MSP) and especially the HeavyMethyl^{™} (HM) technology (Cottrell et al., A real-time PCR assay for DNA-methylation using methylation-specific blockers. Nucleic Acids Res. 2004 Jan 13;32(1):e10).

Another difficulty is the presence of methylated non-tumor DNA in the background DNA of liquid biopsies that often occurs, depending on marker and tumor to be detected. Unless this background is very low or most desirably non-existent, it can be difficult to distinguish between healthy subjects and those having cancer, because the amount of methylated tumor DNA in liquid biopsies is usually very low. So far this second difficulty has been accepted as given by marker and tumor and addressed mostly by increasing specificity, which comes with a loss of sensitivity of detection. The present inventors have, however, focused on the methylation markers in liquid biopsies of healthy subjects instead of primarily cancer patients to increase sensitivity and specificity of cancer detection. To this end, they have, rather than screening for markers methylated in samples from tumor patients, screened for potential cancer markers which are not methylated in liquid biopsies of healthy subjects, to achieve a minimal or non-existent amount of methylated non-tumor DNA in the samples. The inventors have found that, surprisingly, methylated genomic DNA of the markers ANKRD13B and FOXF2 is not or virutally not found in blood or blood-derived samples of healthy subjects, and that these markers show a clear methylation signal in cancer tissues. This property is very rare for a marker and allows for an unusually sensitive and specific detection. Also, the inventors identified a variety of cancers which can be dectected with these markers. Methods for detecting cancer which are adapted according to these findings will allow for an improved care for cancer patients by providing the possibility of the most promising time window for treatment.

### SUMMARY OF THE INVENTION

In a first aspect, the present invention relates to a method for detecting DNA methylation within genomic DNA from a sample comprising cell-free DNA from blood or a sample derived therefrom of a subject, wherein the genomic DNA has a sequence comprised in SEQ ID NO: 21 and comprises at least 4 CpG dinucleotides, and wherein the subject has an increased risk for or is suspected of having a cancer selected from the group consisting of stomach, liver, colon, ovary, esophagus, bladder and prostate cancer.

In a second aspect, the present invention relates to a method for detecting the presence or absence of cancer or a risk thereof in a subject, comprising detecting DNA methylation within genomic DNA from a sample comprising cell-free DNA from blood or a sample derived therefrom of a subject, wherein the genomic DNA has a sequence comprised in SEQ ID NO: 21 and comprises at least 4 CpG dinucleotides, and wherein the cancer is selected from the group consisting of stomach, liver, colon, ovary, esophagus, bladder and prostate cancer.

In a third aspect, the present invention relates to the use of an oligonucleotide selected from the group consisting of a primer, blocker or probe, which has a sequence that is substantially identical or complementary to a stretch of contiguous nucleotides of SEQ ID NOs 22-25, in a method for detecting DNA methylation for the diagnosis of cancer, for the assessment of the response to treatment of cancer, for the monitoring of cancer, or for the screening of subjects to detect an increased likelihood of cancer, wherein the method comprises detecting DNA methylation according to the first aspect, and wherein the cancer is selected from the group consisting of stomach, liver, colon, ovary, esophagus, bladder and prostate cancer.

In a fourth aspect, the present invention relates to the use of a kit comprising at least a first and a second oligonucleotide, wherein the first oligonucleotide and the second oligonucleotide are oligonucleotides as defined in the fourth aspect, in a method for detecting DNA methylation for the diagnosis of cancer, for the assessment of the response to treatment of cancer, for the monitoring of cancer, or for the screening of subjects to detect an increased likelihood of cancer, wherein the method comprises detecting DNA methylation according to the first aspect, and wherein the cancer is selected from the group consisting of stomach, liver, colon, ovary, esophagus, bladder and prostate cancer.

### LEGENDS TO THE FIGURES

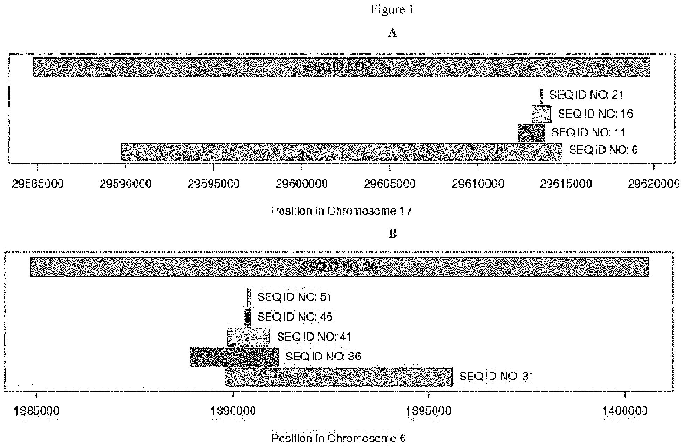

**Figure 1****:** Map of preferred target regions. A: ANKRD13B, B: FOXF2. See Table 1 for an explanation of the SEQ ID NOs.

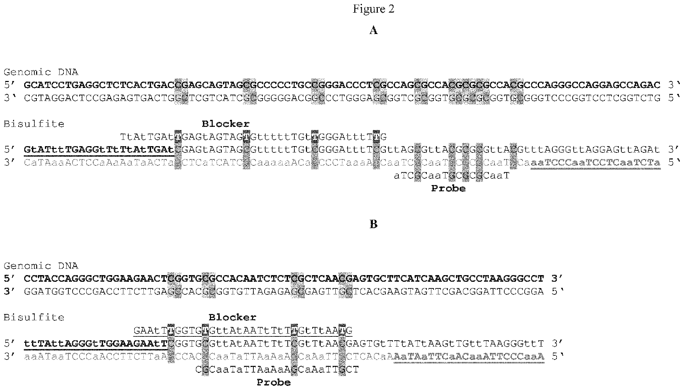

**Figure 2****:** Assay examples. A: ANKRD13B, B: FOXF2. DNA sequences are in the sense orientation of the GRCh38 genome build. Assays and their elements are aligned with the genomic reference sequence (genomic, both strands shown) on the top. Below the genomic sequence the bisulfite and PCR converted sense strand of the genomic DNA is shown, wherein C in non-CpG context is converted to T (bisulfite converts to U, which pairs with A and is converted to T by amplification). The bisulfite strand is the initial template for amplification. The reverse complement strand shown in grey below is unavailable until it is synthesized by amplification. All DNA single strand sequences are annotated with 3' and 5' on both ends according to their orientation. Primer sequences are shown by underlined sequences. Other oligomers like blockers and probes are shown in alignment with the assay and genomic sequences. Cytosine in CpG context, their counterparts on the opposite strand and/or any base in probe or blocker covering them indirectly or directly are shown with grey background. Capital letters are used in different context: For all genomic reference sequences and for the bases in other strands that are not converted and are identical to the genomic sequence. Lower case letters show converted bases.

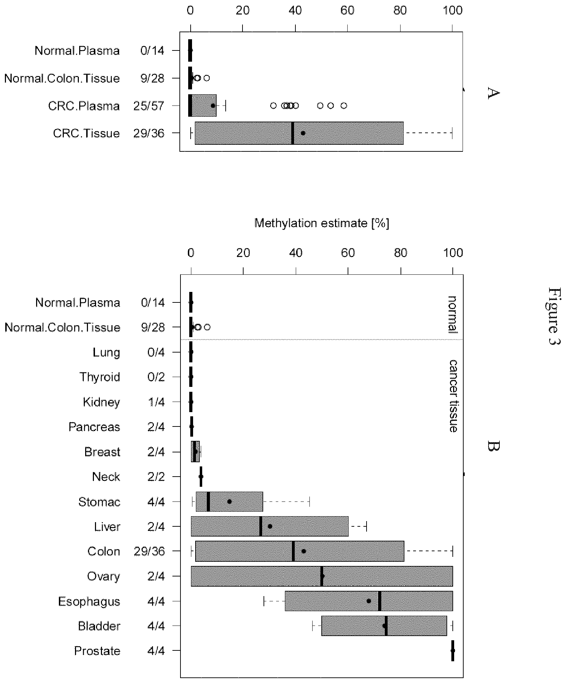

**Figure 3****:** Boxplots of DNA methylation measured with the ANKRD13B assay on different kinds of samples. Numbers below the x-axis provide information how many PCR reactions were done and how many of them lead to any signal (>0%), which is important information for a qualitative analysis of the data. Mean methylation is shown as dark points. Boxplot A provides example information for the diagnostic value in colorectal cancer (CRC): The marker showed no signal in plasma from normal patients, very few and low signals in normal colon tissue but high signals in most colon cancer tissue and was positive in 44% of the PCR reactions on blood plasma from cancer patients. Boxplot B shows two normal and 13 cancer tissue types. It provides evidence that the ANKRD13B marker is a cancer marker that is usable as a diagnostic and/or prognostic marker in stomach, liver, colon, ovary, esophagus, bladder and prostate cancer.

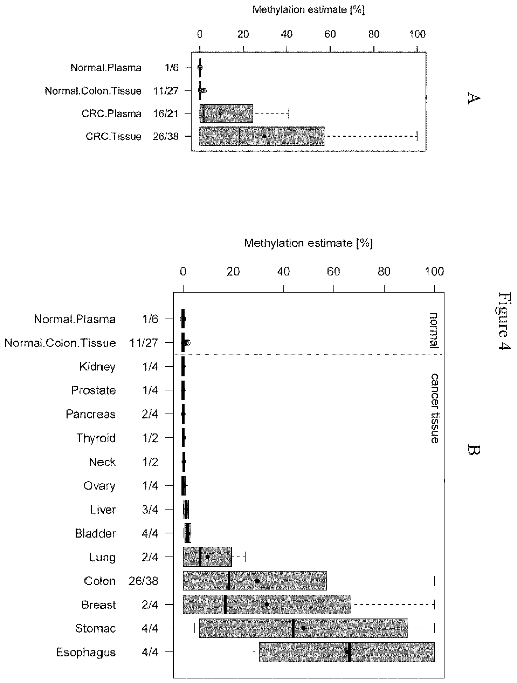

**Figure 4****:** Boxplots of DNA methylation measured with the FOXF2 assay on different kinds of samples. Mean methylation is shown as dark points. Numbers below the x-axis provide information how many PCR reactions were done and how many of them lead to any signal (>0%), which is important information for a qualitative analysis of the data. Mean methylation is shown as dark points. Boxplot A provides example information for the diagnostic value in colorectal cancer (CRC): The marker showed virtually no signal in plasma from normal patients, very few and low signals in normal colon tissue but high signals in most colon cancer tissue and was positive in 76% of the PCR reactions on blood plasma from cancer patients. Boxplot B shows two normal and 13 cancer tissue types. It provides evidence that the FOXF2 marker is a cancer marker that is usable as a diagnostic and/or prognostic marker in lung, colon, breast, stomach and esophagus cancer.

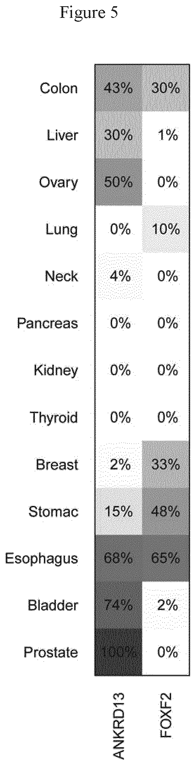

**Figure 5****:** Average methylation [%] of the two cancer markers ANKRD13B and FOXF2 measured over tissue samples from different cancer types. The clustering of the two markers demonstrates in how far a paneling can be of use to diagnose cancer and specify the organ of origin for a series of different cancers in parallel.

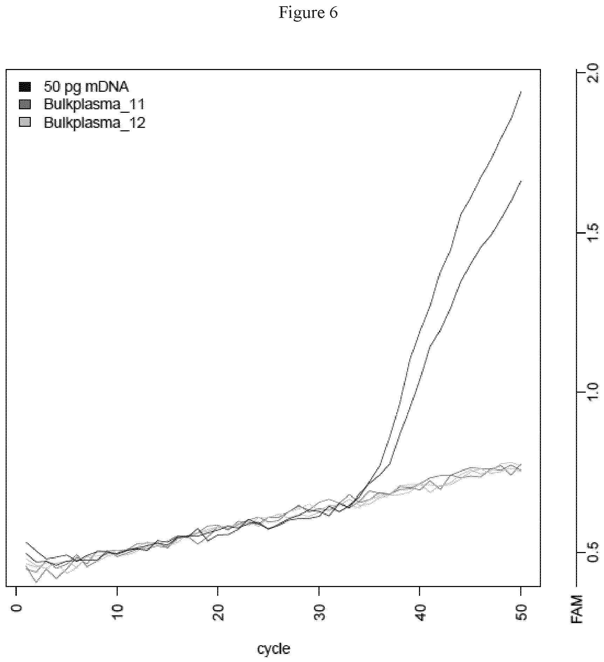

**Figure 6****:** ANKRD13B assay realtime PCR amplification curves for bulk plasma (pooled plasma) from healthy individuals in comparison to a sample with very low amount of methylated DNA (50 pg).

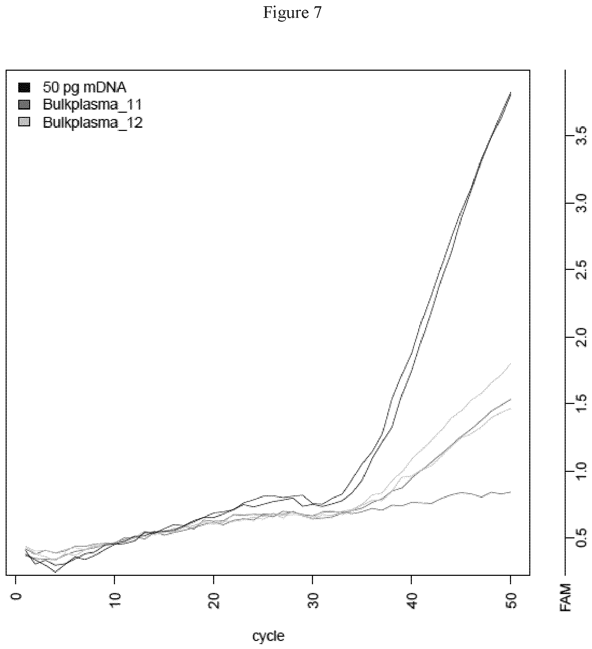

**Figure** 7: FOXF2 assay realtime PCR amplification curves for bulk plasma from healthy individuals in comparison to a sample with very low amount of methylated DNA (50 pg).

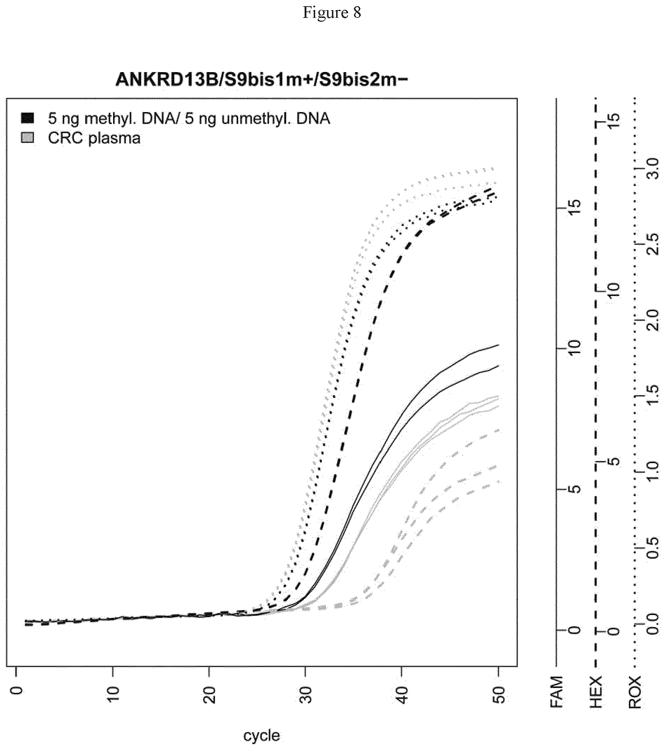

**Figure 8****:** ANKRD13B triplex assay amplification curves for CRC plasma in grey (and a calibrator in black). The dotted curves show the unmethylated background DNA as determined by unmethylated Septin9, the dashed curves show CRC tumor DNA as determined by methylated Septin9. The straight lines show methylation measured for ANKRD13B.

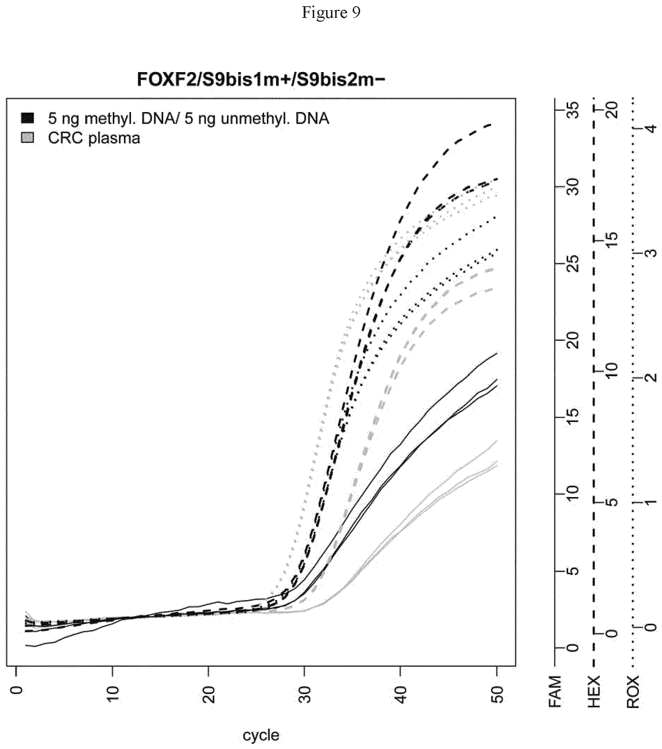

**Figure** 9: FOXF2 triplex assay amplification curves for CRC plasma in grey (and a calibrator in black). The dotted curves show the unmethylated background DNA as determined by unmethylated Septin9, the dashed curves show CRC tumor DNA as determined by methylated Septin9. The straight lines show methylation measured for FOXF2.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Nothing herein is to be construed as an admission that the invention is not entitled to antedate such disclosure by virtue of prior invention.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

### Aspects of the invention and particular embodiments thereof

In a first aspect, the present invention relates to a method for detecting DNA methylation within genomic DNA from a sample comprising cell-free DNA from blood or a sample derived therefrom of a subject, wherein the genomic DNA has a sequence comprised in SEQ ID NO: 21 and comprises at least 4 CpG dinucleotides, and wherein the subject has an increased risk for or is suspected of having a cancer selected from the group consisting of stomach, liver, colon, ovary, esophagus, bladder and prostate cancer.

In a preferred embodiment, the cancer selected from the group consisting of stomach, liver, colon, ovary, esophagus, bladder and prostate cancer is as as defined by the American Cancer Society. The cancer may be of any subtype and stage as defined below.

Specifically, it is preferred that when DNA methylation with respect to SEQ ID NO: 21 is detected for diagnosing or screening for cancer, one or more of the following risk factors can be attributed to the subject:
- Stomach cancer: One or more risk factors selected from the group consisting of male gender, age of 50 or older, ethnicity (in particular Hispanic American, African American or Asian/Pacific Islander), Helicobacter pylori infection, stomach lymphoma, diet rich of smoked foods, salted fish and meat and/or pickled vegetables, tobacco consumption (in particular smoking), being overweight or obese, previous stomach surgery, pernicious anemia, Menetrier disease (hypertrophic gastropathy), type A blood, inherited cancer syndromes or predispositions (including hereditary diffuse gastric cancer, hereditary non-polyposis colorectal cancer (HNPCC), familial adenomatous polyposis (FAP), BRCA1 and/or BRCA2 mutations, Li-Fraumeni syndrome, Peutz-Jeghers syndrome (PJS), family history of stomach cancer (first degree relative, i.e. parents, siblings or children), non-cancerous stomach polyps, Epstein-Barr virus infection, occupation (in particular workers in the coal, metal and rubber industries), and common variable immune deficiency (CVID).
- Liver cancer: One or more risk factors selected from the group consisting of age of 45 or older (in particular 50 or older), male gender, ethnicity (in particular Asian/Pacific Islander), chronic viral hepatitis, cirrhosis, heavy alcohol use (more than 3 or 4 alcohol units a day for men, or more than 2 or 3 alcohol units a day for women; an alcohol unit is defined as 10 ml (8 g) of pure alcohol), obesity, type 2 diabetes, inherited metabolic diseases (in particular those leading to cirrhosis, such as hereditary hemochromatosis, tyrosinemia, alpha1-antitrypsin deficiency, porphyria cutanea tarda, glycogen storage diseases, Wilson disease), exposure to a carcinogen (in particular aflatoxins, vinyl chloride and thorium dioxide (Thorotrast), anabolic steroids, arsenic, infection with parasites (in particular those causing schistosomiasis), and tobacco consumption (in particular smoking).
- Colon cancer: One or more risk factors selected from the group consisting of age of 50 or older, a personal history of colorectal polyps or colorectal cancer (wherein the cancer was completely removed), a personal history of inflammatory bowel disease, family history of colorectal cancer or adenomatous polyps (first degree relative), having an inherited syndrome (such as familial adenomatous polyposis, Lynch syndrome (hereditary non-polyposis colon cancer, or HNPCC), Turcot syndrome, Peutz-Jeghers syndrome, or MUTYH-associated polyposis), ethnicity (in particular African American or Jew of Eastern European descent), having type 2 diabetes, being overweight or obese, physical inactivity, tobacco consumption (in particular smoking), and heavy alcohol use (as defined above).
- Ovary cancer: One or more risk factors selected from the group consisting of age of 40 or older (in particular of 63 and older and more particular after menopause), female gender, obesity, first full-term pregnancy after age 35 or no pregancy, no use of birth control pills (in particular never or not in the last 3, 4 or 6 months), exposure to fertility drugs (in particular clomiphene citrate), exposure to androgens, estrogen therapy and/or hormone therapy, family history of ovarian cancer, breast cancer, or colorectal cancer (in particular in first degree relatives), having an inherited cancer syndrome or predisposition (such as hereditary breast and ovarian cancer syndrome, PTEN tumor hamartoma syndrome, hereditary nonpolyposis colon cancer, Peutz-Jeghers syndrome, or MUTYH-associated polyposis), personal history of breast cancer, use of talcum powder applied directly to the genital area, high fat diet, use of analgesics, and tobacco consumption (in particular smoking).
- Esophagus cancer: One or more risk factors selected from the group consisting of male gender, age of 50 or older (in particular 55 or older), gastroesophageal reflux disease, Barrett's esophagus, tobacco consumption (in particular smoking), heavy alcohol use (as defined above), being overweight or obese, diet rich in processed meats, drinking hot liquids regularly, achalasia, tylosis, Plummer-Vinson syndrome, occupation (in particular work exposure to chemical fumes, e.g. solvents used for dry cleaning), past injury to the esophagus, personal history of other cancers (such as lung cancer, mouth cancer, or throat cancer), and human papilloma virus (HPV) infection.
- Bladder cancer: One or more risk factors selected from the group consisting of tobacco consumption (in particular smoking), occupation (in particular work exposure to aromatic amines and/or workers in rubber, leather, textiles, paint and printing industries as well as machinists, hairdressers and truck drivers), ethnicity (in particular White), age of 50 or older (in particular 55 or older), male gender, chronic bladder irritation and infections, personal history of bladder or other urothelial cancer (completely removed and/or other subtype), bladder birth defects, family history of bladder cancer (in particular in first degree relatives), having an inherited syndrome or predisposition (such as a mutation of the retinoblastoma (RB1) gene, Cowden disease caused by mutations in the PTEN gene, or Lynch syndrome), past or current chemotherapy with cyclophosphamide, past or current radiation therapy with radiation to the pelvis, exposure to pioglitazone, food supplements containing aristolochic acid and/or arsenic in drinking water, and low fluid consumption.
- Prostate cancer: One or more risk factors selected from the group consisting of age of 50 or older (in particular 65 or older), male gender, ethnicity (in particular African American and Caribbean men of African ancestry), family history of prostate cancer (in particular in first degree relatives), having an inherited syndrome or predisposition (such as a mutation of the BRCA1 or BRCA2 gene, or Lynch syndrome), diet (in particular rich in red meat or high-fat dairy products), obesity, tobacco consumption (in particular smoking), occupation (in particular firefighters), inflammation of the prostate, sexually transmitted infections (in particular gonorrhea or chlamydia), and vasectomy.

Further, it is preferred that when additionally DNA methylation with respect to SEQ ID NO: 26 is detected for diagnosing or screening for a cancer selected from the group consisting of lung, colon, breast, stomach and esophagus cancer, one or more of the following risk factors can be attributed to the subject:
- Lung cancer: One or more risk factors selected from the group consisting of tobacco consumption (in particular smoking), exposure to radon, exposure to asbestos, exposure to radioactive ores such as uranium, inhalation of chemicals or minerals such as arsenic, beryllium, cadmium, silica, vinyl chloride, nickel compounds, chromium compounds, coal products, mustard gas, or chloromethyl ethers, inhalation of diesel exhaust, increased exposure to air pollution (e.g. due to working and/or living in inner cities), radiation therapy to the lungs, arsenic in drinking water, personal history of lung cancer (completely removed and/or other subtype), and family history of lung cancer (in particular first degree relatives).
- Colon cancer: One or more risk factors selected from the group defined above with respect to SEQ ID NO: 1.
- Breast cancer: One or more risk factors selected from the group consisting of female gender, age of 45 or older (in particular 50 or 55 or older), having a risk-increasing inherited syndrome or predisposition (such as mutation of the BRCA1, BRCA2, ATM, TP53, CHEK2, PTEN, CDH1, STK11 and/or PALB2 gene), family history of breast cancer (in particular first degree relatives), personal history of breast cancer in another part of the breast or in the other breast, ethnicity (in particular White), dense breast tissue (in particular a dense breast on mammogram 1.2 to 2 times of the average breast density of women), benign breast conditions (such as (i) non-proliferative lesions including fibrosis and/or simple cysts, mild hyperplasia, adenosis (non-sclerosing), ductal ectasia, Phyllodes tumor (benign), single papilloma, fat necrosis, periductal fibrosis, squamous and apocrine metaplasia, and epithelial-related calcifications, or (ii) proliferative lesions without atypia including usual ductal hyperplasia (without atypia), fibroadenoma, sclerosing adenosis, papillomas (in particular papillomatosis), and radial scar, or (iii) proliferative lesions with atypia including atypical ductal hyperplasia (ADH) and atypical lobular hyperplasia (ALH)), lobular carcinoma in situ, above average number of menstrual periods (e.g. due to menstruation before age of 12 and/or menopause after age of 55), previous chest radiation, diethylstilbestrol exposure, not having children or having the first child after the age of 30 (in particular combined with the age risk factor defined above), use of oral contraceptives or depot-medroxyprogesterone acetate, hormone therapy after menopause, heavy alcohol use (as defined above), being overweight or obese, and physical inactivity.
- Stomach cancer: One or more risk factors selected from the group defined above with respect to SEQ ID NO: 1.
- Esophagus cancer: One or more risk factors selected from the group defined above with respect to SEQ ID NO: 1.

The genomic DNA comprises at least 4, 5, or 6 CpG dinucleotides. Generally, the methylation of at least 4, 5, or 6 CpG dinucleotides comprised in the genomic DNA is detected.

Further, it is preferred that the genomic DNA having a sequence comprised in SEQ ID NO: 26 has a sequence comprised in SEQ ID NO: 31 and/or 36, preferably in SEQ ID NO: 41, more preferably in SEQ ID NO: 46 and most preferably in SEQ ID NO: 51.

Definitions and embodiments described below, in particular under the header 'Definitions and further embodiments of the invention' apply to the method of the first aspect.

In a second aspect, the present invention relates to a method for detecting the presence or absence of cancer or a risk thereof in a subject, comprising detecting DNA methylation within genomic DNA from a sample comprising cell-free DNA from blood or a sample derived therefrom of a subject, wherein the genomic DNA has a sequence comprised in SEQ ID NO: 21 and comprises at least 4 CpG dinucleotides, and wherein the cancer is selected from the group consisting of stomach, liver, colon, ovary, esophagus, bladder and prostate cancer.

The cancer may be of any subtype and stage as defined below, i.e. the presence or absence of any subtype and/or stage can be detected.

In a preferred embodiment,
(i) the presence of a significant amount of methylated genomic DNA is indicative for the presence of stomach, liver, colon, ovary, esophagus, bladder or prostate cancer or a risk thereof, and the absence of a significant amount of methylated genomic DNA is indicative for the absence of stomach, liver, colon, ovary, esophagus, bladder and prostate cancer or a risk thereof.

The term "a significant amount of methylated genomic DNA" as used herein refers to an amount of at least X molecules of the methylated target DNA per ml of the sample used, preferably per ml of blood, serum or plasma. X may be as low as 1 and is usually a value between and including 1 and 50, in particular 2, 3, 4, 5, 10, 15, 20, 25, 30 or 40. For determination whether there is such a significant amount, the methylated target DNA may be, but does not necessarily have to be quantified. The determination, if no quantification is performed, may also be made by comparison to a standard, for example a standard comprising genomic DNA and therein a certain amount of fully methylated DNA, e.g. the equivalence of X genomes, wherein X is as above. The term may also refer to an amount of at least Y% of methylated target DNA in the sample (wherein the sum of methylated and unmethylated target DNA is 100%), wherein Y may be as low as 0.05 and is usually a value between and including 0.05 and 5, preferably 0.05 and 1 and more preferably 0.05 and 0.5. For example, Y may be 0.05, 0.1, 0.2, 0.3, 0.5, 1.0, 1.5, 2.0, 2.5, 3.0, 4.0 or 5.0.

A preferred way of carrying out the method of the first or second aspect comprises the steps of
(a) converting cytosine unmethylated in the 5-position to uracil or another base that does not hybridize to guanine in the genomic DNA;
(b) amplifying methylation-specifically a region of the converted DNA;
(c) detecting the presence or absence of DNA amplified in step (b);
wherein the presence or absence of amplified DNA is indicative of the presence or absence, respectively, of methylated genomic DNA.

The amplification is preferably performed by methylation-specific PCR (i.e. an amplicon is produced depending on whether one or more CpG sites are converted or not) using primers which are methylation-specific (and specific to bisulfite-converted DNA) or more preferably primers which are methylation-unspecific, but specific to bisulfite-converted DNA (i.e. hybridize only to converted DNA by covering at least one converted C). In case of the latter, methylation-specificity is achieved by using methylation-specific blocker oligonucleotides, which hybridize specifically to converted or non-converted CpG sites and thereby terminate the PCR polymerization. In a most preferred embodiment, the step of amplifying comprises a PCR, preferably a real-time PCR, in particular HeavyMethyl^{™} or HeavyMethyl^{™}-MethyLight^{™}.

In other words, the region of the converted DNA is amplified methylation-specifically by using at least one methylation-specific oligonucleotide. Preferably, the at least one methylation-specific oligonucleotide is a primer or a blocker. In specific embodiments, the region of the converted DNA is amplified methylation-specifically by using one or two (i.e. a pair of) methylation-specific primers, or by using two (i.e. a pair of) methylation-unspecific primers and one or two (i.e. pair of) methylation specific blockers.

Further, the at least one methylation-specific oligonucleotide (e.g. a primer, blocker or a probe as referred to herein) preferably is substantially identical or complementary to a stretch of contiguous nucleotides of SEQ ID NOs 22-25.

The presence or absence of amplified DNA can be detected as described below, in particular by real-time PCR or by Next Generation Sequencing

In a preferred embodiment, detecting the presence of absence of DNA amplified in step (b) comprises determining the amount of DNA amplified in step (b). The amount can be determined with the detection means described below, in particular by real-time PCR or by Next Generation Sequencing.

It is preferred that additionally, converted DNA is amplified methylation-specifically by using at least one methylation-specific oligonucleotide which is substantially complementary to a stretch of contiguous nucleotides of
- SEQ ID NOs 32-35 for amplifying converted DNA derived from genomic DNA having a sequence comprised in SEQ ID NO: 31,
- SEQ ID NOs 37-40 for amplifying converted DNA derived from genomic DNA having a sequence comprised in SEQ ID NO: 36,
- SEQ ID NOs 42-45 for amplifying converted DNA derived from genomic DNA having a sequence comprised in SEQ ID NO: 41,
- SEQ ID NOs 47-50 for amplifying converted DNA derived from genomic DNA having a sequence comprised in SEQ ID NO: 46, and
- SEQ ID NOs 52-55 for amplifying converted DNA derived from genomic DNA having a sequence comprised in SEQ ID NO: 51.

In a particular embodiment, the method of the second aspect further comprises confirming an indicated cancer and/or narrowing down the indicated cancers by using one or more further means for detecting an indicated cancer. The further means may be a cancer marker (or "biomarker") or a conventional (non-marker) detection means. The cancer marker can for example be a DNA methylation marker, a mutation marker (e.g. SNP), an antigen marker, a protein marker, a miRNA marker, a cancer specific metabolite, or an expression marker (e.g. RNA or protein expression). The conventional means can for example be a biopsy (e.g. visual biopsy examination with or without staining methods for example for protein or expression markers), an imaging technique (e.g. X-ray imaging, CT scan, nuclear imaging such as PET and SPECT, ultrasound, magnetic resonance imaging (MRI), thermography, endoscopy, digital mammography, colonoscopy or virtual colonoscopy, laparoscopy, angiogram, bone scan or sentinel node mapping for breast cancer) or a physical, e.g. tactile examination. If the further means is for confirmation of a single indicated cancer, it is preferred that it is a biopsy or other means that removes and examines a solid tissue sample of the subject from the tissue for which the cancer is indicated (i.e. no liquid tissue such as blood). If the further means is for narrowing down a set of indicated cancers, it is preferred that it is a non-invasive or a minimally invasive means. Such means can be a further marker or a conventional means such as an imaging technique or a physical examination. Generally, the further means is suitable for detecting a cancer the presence of which is indicated by the presence of DNA methylation of genomic DNA having a sequence comprised in SEQ ID NO: 21 and comprising at least 4 CpG dinucleotides. Specifically, it is preferred that
- for stomach cancer, the further means for cancer detection is selected from the group consisting of biopsy, endoscopy, ultrasound, X-ray, CT scan, MRI, PET scan and laparoscopy;
- for liver cancer, the further means for cancer detection is selected from the group consisting of physical examination, blood test for alphafetoprotein (AFP), ultrasound, CT scan, MRI, angiogram, biopsy, and laparoscopy;
- for colon cancer, the further means for cancer detection is selected from the group consisting of colonoscopy, blood test for anemia and/or carcinoembryonic antigen (CEA), CR scan, MRI, ultrasound, X-ray, and PET scan;
- for ovary cancer, the further means for cancer detection is selected from the group consisting of physical examination, ultrasound, CT scan, X-ray, MRI, PET scan, laparoscopy, biopsy, blood test for CA-125, human chorionic gonadotropin (HCG), alpha-fetoprotein (AFP), lactate dehydrogenase (LDH), inhibin, estrogen and/or testosterone, and colonoscopy;
- for esophagus cancer, the further means for cancer detection is selected from the group consisting of X-ray, upper endoscopy, endoscopic ultrasound, bronchoscopy, biopsy, CT scan, MRI and PET scan;
- for bladder cancer, the further means for cancer detection is selected from the group consisting of cytoscopy, urine test for tumor cells, biopsy (in particular transurethral resection of bladder tumor), CT scan, MRI and PET scan;
- for prostate cancer, the further means for cancer detection is selected from the group consisting of PSA test, DRE test, PCA3 test, ultrasound (in particular transrectal ultrasound), biopsy, MRI, MRI fusion biopsy, and CT scan;
- for lung cancer, the further means for cancer detection is selected from the group consisting of biopsy, testing for mutations in on one or more of the genes EGFR, ALK, KRAS, BRAF, HER2, ROS1, and/or RET (in particular EGFR and/or ALK), sputum cytology, bronchoscopy, needle aspiration or core biopsy, thoracentesis, thoracoscopy, mediastinoscopy, thoracotomy, endobronchial ultrasound, endoscopic esophageal ultrasound, CT scan, PET scan, and MRI scan;
- for breast cancer, the further means for cancer detection is selected from the group consisting of mammography, ultrasound, MRI, biopsy, complete blood count, serum chemistry, and physical examination.

In a preferred embodiment, the further means is suitable for detecting
stomach, liver, colon, ovary, esophagus, bladder and/or, preferably or, prostate cancer or a risk thereof for confirming and/or narrowing down the determination of the presence of cancer using genomic DNA having a sequence comprised in SEQ ID NO: 21 and comprising at least 4 CpG dinucleotides

Definitions given and embodiments described with respect to the first aspect apply also to the second aspect, in as far as they are applicable. Also, definitions and embodiments described below, in particular under the header 'Definitions and further embodiments of the invention' apply to the method of the second aspect.

Described is an oligonucleotide selected from the group consisting of a primer, blocker or probe, which has a sequence that is substantially identical or complementary to a stretch of contiguous nucleotides of SEQ ID NOs 2-5 or SEQ ID NOs 27-30.

Preferably, the oligonucleotide has a sequence that is substantially identical or complementary to a stretch of contiguous nucleotides of a SEQ ID NO selected from the group consisting of SEQ ID NOs 7-10, 12-15, 17-20 and 22-25, or from the group consisting of SEQ ID NOs 32-35, 37-40, 42-45, 47-50 and 52-55.

Generally, the oligonucleotide comprises at least 1, 2 or 3 dinucleotides derived from conversion of the C of a CpG dinucleotide in SEQ ID NO: 1 (alternatively SEQ ID NO: 26 or a sub-sequence of either as recited above) or its complement into a T. In this case, the oligonucleotide is a methylation-specific oligonucleotide. This methylation-specific oligonucleotide is also specific for bisulfite-converted DNA, since it comprises at least one nucleotide derived from conversion of a C not in a CpG context (e.g. of a CpC, CpA or CpT dinucleotide) in SEQ ID NO: 1 (alternatively SEQ ID NO: 26 or a sub-sequence of either as recited above) or its complement into a T.

Alternatively, the oligonucleotide may also not comprise a dinucleotide derived from conversion of the C of a CpG dinucleotide in SEQ ID NO: 1 (alternatively SEQ ID NO: 26 or a sub-sequence of either as recited above) or its complement into a T. In that case, it is a methylation-unspecific oligonucleotide, which is nonetheless specific for bisulfite-converted DNA, since it comprises at least one nucleotide derived from conversion of a C not in a CpG context (e.g. a C of a CpC, CpA or CpT dinucleotide) in SEQ ID NO: 1 (alternatively SEQ ID NO: 26 or a sub-sequence of either as recited above) or its complement into a T. A methylation-unspecific oligonucleotide can, however, cover one or more dinucleotides derived from conversion of the C of a CpG dinucleotide in SEQ ID NO: 1 (alternatively SEQ ID NO: 26 or a sub-sequence of either as recited above) or its complement into a T with a mismatch that does not distinguish between converted and not converted C or with a spacer as defined herein.

A probe preferably has one or more modifications selected from the group consisting of a detectable label and a quencher, and/or a length of 5-40 nucleotides. Further, a probe is preferably a methylation-specific oligonucleotide. A blocker preferably has one or more modifications selected from the group consisting of a non-extensible 3' end and a backbone resistant to the 5' nuclease activity of polymerase, and/or a length of 15-50 nucleotides. Further, a blocker is preferably a methylation-specific oligonucleotide. A primer preferably has a length of 10-40 nucleotides. In particular the primer may be either a methylation-specific oligonucleotide or a methylation-unspecific oligonucleotide.

The blocker may have a sequence according to SEQ ID NO: 58 with a 3' and/or 5' deletion or addition of up to 5 nucleotides and/or the probe has a sequence according to SEQ ID NO: 59 with a 3' and/or 5' deletion or addition of up to 5 nucleotides, wherein the additions preferably are according to SEQ ID NO: 1 (i.e. are extensions of the blocker in SEQ ID NO: 1). In another specific embodiment, the blocker has a sequence according to SEQ ID NO: 62 with a 3' and/or 5' deletion or addition of up to 5 nucleotides and/or the probe has a sequence according to SEQ ID NO: 63 with a 3' and/or 5' deletion or addition of up to 5 nucleotides, wherein the additions preferably are according to SEQ ID NO: 26 (i.e. are extensions of the blocker in SEQ ID NO: 26).

Definitions given and embodiments described with respect to the first and second aspect apply also to the described oligonulceotide, in as far as they are applicable. Also, definitions and embodiments described below, in particular under the header 'Definitions and further embodiments of the invention' apply to the described oligonucleotide.

Described is a kit comprising at least a first and a second oligonucleotide, wherein the first and the second oligonucleotides are oligonucleotides of aspect 3.

In a kit wherein the oligonucleotides are suitable for a real-time PCR, it is preferred that the first oligonucleotide is a methylation-specific probe according to aspect 3 and the second oligonucleotide is a methylation-specific blocker or a methylation-specific primer, according to aspect 3. In a preferred embodiment, the second oligonucleotide is a methylation-specific blocker. In this embodiment, it is preferred that the kit further comprises a methylation-unspecific primer according to aspect 3.

In a kit wherein the oligonucleotides are suitable for a PCR follow by sequencing, the first oligonucleotide is a primer according to aspect 3 and the second oligonucleotide is also a primer according to aspect 3 or a methylation-specific blocker according to aspect 3. In one embodiment, the first oligonucleotide and the second oligonucleotide are methylation-unspecific primers. Preferably, these primers are a primer pair, i.e. a forward and a reverse primer. This kit is for the simultaneously amplification and sequencing of both methylated and unmethylated target DNA (the latter being for example non-tumor background DNA), wherein the amount of unmethylated target DNA is determined for quantification of the methylated target DNA as described herein. In another embodiment, the first oligonucleotide and the second oligonucleotide are methylation-specific primers. Preferably, these primers are a primer pair, i.e. a forward and a reverse primer. In yet another embodiment, the first oligonucleotide is a methylation-unspecific primer and the second oligonucleotide is a methylation-specific blocker. These kits are for the amplification and sequencing only of the methylated target DNA. Generally, it is preferred that if the kit comprises a primer and/or a blocker, the primer and/or blocker comes as a pair, i.e. as a forward with a reverse primer (or vice versa) and/or a forward with reverse blocker (or vice versa). A forward blocker is a blocker that blocks extension from a forward primer and a reverse blocker is a blocker that blocks extension from a reverse primer.

Definitions given and embodiments described with respect to the first, second and third aspect apply also to the described kit, in as far as they are applicable. Also, definitions and embodiments described below, in particular under the header 'Definitions and further embodiments of the invention' apply to the described kit.

In a third aspect, the present invention relates to the use of an oligonucleotide selected from the group consisting of a primer, blocker or probe, which has a sequence that is substantially identical or complementary to a stretch of contiguous nucleotides of SEQ ID NOs 22-25, in a method for detecting DNA methylation for the diagnosis of cancer, for the assessment of the response to treatment of cancer, for the monitoring of cancer, or for the screening of subjects to detect an increased likelihood of cancer, wherein the method comprises detecting DNA methylation according to the first aspect, and wherein the cancer is selected from the group consisting of stomach, liver, colon, ovary, esophagus, bladder and prostate cancer.

Definitions given and embodiments described with respect to the first and second aspect apply also to the third aspect, in as far as they are applicable. Also, definitions and embodiments described below, in particular under the header 'Definitions and further embodiments of the invention' apply to the use of the third aspect.

In a fourth aspect, the present invention relates the use of a kit comprising at least a first and a second oligonucleotide, wherein the first oligonucleotide and the second oligonucleotide are oligonucleotides as defined in the fourth aspect, in a method for detecting DNA methylation for the diagnosis of cancer, for the assessment of the response to treatment of cancer, for the monitoring of cancer, or for the screening of subjects to detect an increased likelihood of cancer, wherein the method comprises detecting DNA methylation according to the first aspect, and wherein the cancer is selected from the group consisting of stomach, liver, colon, ovary, esophagus, bladder and prostate cancer. Described is the use of a cancer treatment suitable for treating a cancer in a subject in which the presence of cancer has been detected according to the method of the second aspect. Also described is a method comprising the method of the second aspect and subsequently referring a subject for such a cancer treatment. Definitions and further embodiments for the cancer treatment regimen are given below.

Definitions given and embodiments described with respect to the first, second and third aspect apply also to the fourth aspect, in as far as they are applicable. Also, definitions and embodiments described below, in particular under the header 'Definitions and further embodiments of the invention' apply to the method of the sixth aspect.

### Definitions and further embodiments of the invention

The specification uses a variety of terms and phrases, which have certain meanings as defined below. Preferred meanings are to be construed as preferred embodiments of the aspects of the invention described herein. As such, they and also further embodiments described in the following can be combined with any embodiment of the aspects of the invention and in particular any preferred embodiment of the aspects of the invention described above.

The term "detecting DNA methylation" as used herein refers to at least qualitatively analysing for the presence or absence of methylated target DNA. Methylation is preferably determined at 4 or more, 5 or more, 6 or more, 10 or more, 15 or more or 30 or more CpG sites of the target DNA. Usually, the CpG sites analysed are co-methylated in cancer, such that also CpG sites of neighbouring DNA are methylated and can be analysed in addition or instead. "At least qualitatively" means that also a quantitative determination of methylated target DNA, if present, can be performed. Such a "determining the amount" can be performed as described herein.

The presence or absence of amplified DNA can be detected by any means known in the art, e.g. autoradiography, silver staining or ethidium bromide staining. Preferably, the presence or absence of DNA amplified in step (b) is detected by real-time PCR or by sequencing the amplified DNA. In particular if 3 or more (e.g. 4 or more or 5 or more) different target DNAs (i.e. markers) are examined in parallel, for example a target DNA within the DNA having a sequence comprised in SEQ ID NO: 21 and comprising at least 4 CpG dinucleotides, a target DNA within the DNA according to SEQ ID NOs 26, and one or more further target DNA, it is preferred that the presence or absence of amplified DNA is detected by sequencing.

In a real-time PCR, the presence of DNA amplified in step (b) is preferably detected by using a methylation-specific oligonucleotide which is a probe. The DNA is preferably amplified methylation-specifically using methylation-specific primers or a methylation-specific blocker with methylation-unspecific primers.

A detection by sequencing is preferably a detection by Next Generation Sequencing. Therein, the converted (e.g. bisulfite converted) methylated target DNA of the sample can be amplified methylation-specifically. Then, the amplified sequences are sequenced and the presence of methylated template is deduced from the presence of sequences or sequence reads derived from the amplified converted target DNA.

Generally, a quantification (e.g. determining the amount of methylated target DNA) may be absolute, e.g. in pg per mL or ng per mL sample, copies per mL sample, number of PCR cycles etc., or it may be relative, e.g. 10 fold higher than in a control sample or as percentage of methylation of a reference control. Determining the amount of methylated target DNA in the sample may comprise normalizing for the amount of total DNA in the sample. Normalizing for the amount of total DNA in the test sample preferably comprises calculating the ratio of the amount of methylated target DNA and (i) the amount of DNA of a reference site or (ii) the amount of total DNA of the target (e.g. the amount of methylated target DNA plus the amount of unmethylated target DNA, the latter preferably measured on the reverse strand. A reference site can be any genomic site and does not have to be a gene. It is preferred that the number of occurrences of the sequence of the reference site is stable or expected to be stable (i.e. constant) over a large population (e.g. is not in a repeat, i.e. in repetitive DNA). The reference site can, for instance be a housekeeping gene such as beta-Actin.

The amount of methylated target DNA in the sample may be expressed as the proportion of the amount of methylated target DNA relative to the amount of methylated target DNA in a reference sample comprising substantially fully methylated genomic DNA. Preferably, determining the proportion of methylated target DNA comprises determining the amount of methylated DNA of the same target in a reference sample, inter sample normalization of total methylated DNA, preferably by using the methylation unspecific measurement of a reference site, and dividing the ratio derived from the test sample by the corresponding ratio derived from the reference sample. The proportion can be expressed as a percentage or PMR as defined below by multiplying the result of the division by 100.

The term "PMR", "Percentage of Methylated Reference", or "Percentage of fully Methylated Reference" describes the degree of methylation and is usually calculated by dividing the gene to reference ratio by the gene to completely methylated reference ratio (obtained, e.g. by CpG methyltransferase, for example *SssI* treatment of the normally unmethylated reference) and multiplying by 100. The determination of the PMR is described in detail in Ogino et al. (JMD May 2006, Vol. 8, No. 2). The amount of methylated target DNA may also be calculated with the ΔΔ*Ct* method by using the real-time PCR cycle threshold (Ct) values for target and a reference site (ref) from samples of subjects and the calibrator (cal) sample (methylated at the target locus) as follows: ΔΔ*Ct* = ((Ct_{target} - Ct_{ref}) - (Ct_{targetcal} - Ct_{refcal})); PMR = 100 * x^{-ΔΔ*Ct*}, wherein x is the assumed PCR efficiency. Generally, the PCR efficiency is assumed to be between 1-3, preferably it is 2 or about 2 (e.g. 1.9 to 2.1).

Furthermore, the amount of methylated target DNA may be determined by sequencing, preferably Next Generation Sequencing. Therein, the converted (e.g. bisulfite converted) target DNA can be amplified methylation-unspecifically, i.e. the target DNA is amplified whether or not it is methylated (in other words whether or not cytosines of the CpG sites are converted). This can be achieved by bisulfite-specific primers which are not methylation-specific. Then, the amplified sequences are sequenced and subsequently counted. The ratio of sequences derived from converted methylated DNA (identified in the sequences by CpG sites) and sequences derived from converted unmethylated DNA is calculated, resulting in a (relative) amount of methylated target DNA.

It is preferred that the amount of methylated target DNA is determined by sequencing rather than by real-time PCR in particular if the number of markers (i.e. target DNAs) examined in parallel is 3 or larger, preferably 4 or larger, and more preferably 5 or larger. Preferably, the amount of methylated DNA is the amount over at least 3, more preferably 4-8, most preferably 6 experimental repetitions or parallel experiments.

The term "Next Generation Sequencing" (NGS, also known as 2^{nd} or 3^{rd} generation sequencing) refers to a sequencing the bases of a small fragment of DNA are sequentially identified from signals emitted as each fragment is re-synthesized from a DNA template strand. NGS extends this process across millions of reactions in a massively parallel fashion, rather than being limited to a single or a few DNA fragments. This advance enables rapid sequencing of the amplified DNA, with the latest instruments capable of producing hundreds of gigabases of data in a single sequencing run. See, e.g., Shendure and Ji, Nature Biotechnology 26, 1135-1145 (2008) or Mardis, Annu Rev Genomics Hum Genet. 2008;9:387-402. Suitably NGS platforms are available commercially, e.g. the Roche 454 platform, the Roche 454 Junior platform, the Illumina HiSeq or MiSeq platforms, or the Life Technologies SOLiD 5500 or Ion Torrent platforms.

The term "calibrator sample" refers to a sample comprising control DNA with known DNA concentration and known target methylation state. The control DNA is preferably, but not necessarily, human DNA that is artificially methylated, preferably substantially fully methylated. In a preferred embodiment, the artificial methylation is achieved by using DNA-Methyltransferases. The DNA itself can be, for example, cell line DNA, plasmid DNA, artificial DNA, or combinations/mixtures thereof.

DNA methylated at the target locus is preferably cell line DNA from one or more cell lines, preferably of those that are well characterized and of which the genomic target methylation state is known and/or of which the target is known to be substantially fully methylated. Substantially fully methylated genomic DNA preferably is DNA, particularly genomic DNA, which has all or substantially all CpG sites methylated. "Substantially all" in this respect means at least 95%, 96%, 97%, 98%, 99%, 99.5% or 99.9%. In a preferred embodiment, the methylation of all or substantially all CpG sites is achieved by treating the DNA with a CpG methyltransferase in a manner that provides for the methylation of all or substantially all CpG sites.

The term "methylated" or "hypermethylated" as used herein refers to a biochemical process involving the addition of a methyl group to the cytosine or adenine DNA nucleotides. DNA methylation at the 5 position of cytosine, especially in promoter regions, can have the effect of reducing gene expression and has been found in every vertebrate examined. In adult non-gamete cells, DNA methylation typically occurs in a CpG site. The term "CpG site" or "CpG dinucleotide", as used herein, refers to regions of DNA where a cytosine nucleotide occurs next to a guanine nucleotide in the linear sequence of bases along its length. "CpG" is shorthand for "C-phosphate-G", that is cytosine and guanine separated by only one phosphate; phosphate links any two nucleosides together in DNA. The "CpG" notation is used to distinguish this linear sequence from the CG base-pairing of cytosine and guanine. Cytosines in CpG dinucleotides can be methylated to form 5-methylcytosine. The term "CpG site" or "CpG site of genomic DNA" is also used with respect to the site of a former (unmethylated) CpG site in DNA in which the unmethylated C of the CpG site was converted to another as described herein (e.g. by bisulfite to uracil). The application provides the genomic sequence of each relevant DNA region as well as the bisulfite converted sequences of each converted strand. CpG sites referred to are always the CpG sites of the genomic sequence, even if the converted sequence does no longer contain these CpG sites due to the conversion. Specifically, methylation in the context of the present invention means hypermethylation. The term "hypermethylation" refers to an aberrant methylation pattern or status (i.e. the presence or absence of methylation of one or more nucleotides), wherein one or more nucleotides, preferably C(s) of a CpG site(s), are methylated compared to the same genomic DNA from a non-cancer cell of the subject or a subject not suffering or having suffered from the cancer the subject is treated for, preferably any cancer (healthy control). In particular, it refers to an increased presence of 5-mCyt at one or a plurality of CpG dinucleotides within a DNA sequence of a test DNA sample, relative to the amount of 5-mCyt found at corresponding CpG dinucleotides within a healthy control DNA sample. Hypermethylation as a methylation status/pattern can be determined at one or more CpG site(s). If more than one CpG site is used, hypermethylation can be determined at each site separately or as an average of the CpG sites taken together. Alternatively, all assessed CpG sites must be methylated such that the requirement hypermethylation is fulfilled.

Methylation is detected in particular in a region of the DNA according to the SEQ ID NO referred to (the "target DNA"). The term "target DNA" as used herein refers to a genomic nucleotide sequence at a specific chromosomal location. In the context of the present invention, it is typically a genetic marker that is known to be methylated in the state of disease (for example in cancer cells vs. non-cancer cells). A genetic marker can be a coding or non-coding region of genomic DNA.

The term "region of the target DNA" or "region of the converted DNA" as used herein refers to a part of the target DNA which is to be analysed. Generally, the region is at least 40, 50, 60, 70, 80, 90, 100, 150, or 200 or 300 base pairs (bp) long and/or not longer than 500, 600, 700, 800, 900 or 1000 bp (e.g. 40-500 or 80-500 bp). In particular, it is a region comprising at least 3, 4, 5, 6, 7, 8, 9, 10, 11, 12, 13, 14 or 15 CpG sites of the genomic DNA. Preferably, these sites are not covered by a primer used for amplification of the region. Preferably, at least 3, 4, 5, 6, 7, 8, 9, 10, 11, 12, 13, 14 or 15 (but not necessarily all, in particular CpG sites covered by a spacer or methylation-unspecific mismatch, e.g. in a primer) of these CpG sites are methylated in the target DNA.

The target DNAs of the invention are given in Figure 1 and Table 1.

The term "sample" as used herein refers to biological material obtained from a subject and comprises genomic DNA from all chromosomes, preferably genomic DNA covering the whole genome. The sample comprises, if a subject has cancer, cell-free genomic DNA (including the target DNA) from cancer cells, preferably circulating genomic DNA from cancer cells.

The term "sample comprising cell-free DNA from blood" as used herein refers to a body fluid sample comprising cell-free DNA from blood. While in a preferred embodiment this sample is blood, the term also comprises other body fluids. For example, urine comprises cell-free DNA from blood.. The term "sample derived from a sample comprising cell-free DNA from blood" as used herein refers to any sample that is derived by *in vitro* processing. For example, if the sample is blood, it is preferred that the sample derived therefore is plasma or serum.

The term "cell-free DNA" as used herein or its synonyms "cfDNA", "extracellular DNA", "circulating DNA" and "free circulating DNA" refers to DNA that is not comprised within an intact cell in the respective body fluid which is the sample or from which the sample is derived, but which is freely circulating in the body liquid sample. Cell-free DNA usually is genomic DNA that is fragmented as described below.

Typically, in samples comprising the target DNA, especially extracellular target DNA, from cancer cells, there is also target DNA from non-cancer cells which is not methylated contrary to the target DNA from cancer cells. Usually, said target DNA from non-cancer cells exceeds the amount from diseased cells by at least 10-fold, at least 100-fold, at least 1,000-fold or at least 10,000-fold. Generally, the genomic DNA comprised in the sample is at least partially fragmented. "At least partially fragmented" means that at least the extracellular DNA, in particular at least the extracellular target DNA, from cancer cells, is fragmented. The term "fragmented genomic DNA" refers to pieces of DNA of the genome of a cell, in particular a cancer cell, that are the result of a partial physical, chemical and/or biological break-up of the lengthy DNA into discrete fragments of shorter length. Particularly, "fragmented" means fragmentation of at least some of the genomic DNA, preferably the target DNA, into fragments shorter than 1,500 bp, 1,300 bp, 1,100 bp, 1,000 bp, 900 bp, 800 bp, 700 bp, 600 bp, 500 bp, 400 bp, 300 bp, 200 bp or 100 bp. "At least some" in this respect means at least 5%, 10%, 20%, 30%, 40%, 50% or 75%.

The term "genomic DNA" as used herein refers to chromosomal DNA and is used to distinguish from coding DNA. As such, it includes exons, introns as well as regulatory sequences, in particular promoters, belonging to a gene.

The phrase "converting, in DNA, cytosine unmethylated in the 5-position to uracil or another base that does not hybridize to guanine" as used herein refers to a process of chemically treating the DNA in such a way that all or substantially all of the unmethylated cytosine bases are converted to uracil bases, or another base which is dissimilar to cytosine in terms of base pairing behaviour, while the 5-methylcytosine bases remain unchanged. The conversion of unmethylated, but not methylated, cytosine bases within the DNA sample is conducted with a converting agent. The term "converting agent" as used herein relates to a reagent capable of converting an unmethylated cytosine to uracil or to another base that is detectably dissimilar to cytosine in terms of hybridization properties. The converting agent is preferably a bisulfite such as disulfite, or hydrogen sulfite. The reaction is performed according to standard procedures (Frommer et al., 1992, Proc Natl Acad Sci USA 89:1827-31; Olek, 1996, Nucleic Acids Res 24:5064-6; EP 1394172). It is also possible to conduct the conversion enzymatically, e.g by use of methylation specific cytidine deaminases. Most preferably, the converting agent is sodium bisulfite or bisulfite.

The term "amplifying" or "generating an amplicon" as used herein refers to an increase in the number of copies of the target nucleic acid and its complementary sequence, or particularly a region thereof. The amplification may be performed by using any method known in the art. The amplification of nucleic acid includes methods that require multiple cycles during the amplification process or method that are performed at a single temperature. Cycling techniques are exemplified by methods requiring thermo-cycling. The methods requiring thermo-cycling include polymerase chain reaction (PCR), which is well known in the art. The PCR includes denaturing a double-stranded DNA into single stranded DNAs by thermal denaturation, annealing a primer to the single stranded DNAs; and synthesizing a complementary strand from the primer. Isothermal amplification is an amplification performed at a single temperature or where the major aspect of the amplification process is performed at a single temperature. In the PCR process, the product of the reaction is heated to separate the two strands such that another primer may bind to the template. Conversely, the isothermal techniques rely on a strand displacing polymerase in order to separate the two strands of a double strand and re-copy the template. Isothermal techniques may be classified into methods that rely on the replacement of a primer to initiate a reiterative template copying and those that rely on continued re-use or new synthesis of a single primer molecule. The methods that rely on the replacement of the primer include helicase dependant amplification (HDA), exonuclease dependent amplification, recombinase polymerase amplification (RPA), and loop mediated amplification (LAMP). The methods that rely on continued re-use or new synthesis of a single primer molecule include strand displacement amplification (SDA) or nucleic acid based amplification (NASBA and TMA).

The amplification is preferably performed by methylation-specific PCR (i.e. an amplicon is produced depending on whether one or more CpG sites are converted or not) using primers which are methylation-unspecific, but specific to bisulfite-converted DNA (i.e. hybridize only to converted DNA by covering at least one converted C). Methylation-specificity is achieved by using methylation-specific blocker oligonucleotides, which hybridize to specifically to converted or non-converted CpG sites and thereby terminate the PCR polymerization. In a most preferred embodiment, the step of amplifying comprises a real-time PCR, in particular HeavyMethyl^{™} or HeavyMethyl^{™}-MethyLight^{™}. Alternatively, a methylation-specific PCR may use methylation-specific primers and no blockers.

The term HeavyMethyl^{™} as used herein refers to an assay, wherein methylation specific blocking probes (also referred to herein as blockers) covering CpG positions between, or covered by the amplification primers enable methylation-specific selective amplification of the target DNA or a region thereof.

The term "MethyLight^{™}" refers to a high-throughput quantitative methylation assay that utilizes fluorescence-based real-time PCR technology that requires no further manipulations after the PCR step (Eads et al., Cancer Res. 59:2302-2306, 1999). Briefly, the MethyLight^{™} process begins with a mixed sample of genomic DNA that is converted, e.g. in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process. It may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. The term "HeavyMethyl^{™} MethyLight^{™}" assay refers to a HeavyMethyl^{™} MethyLight^{™} assay, which is a variation of the MethyLight^{™} assay, wherein the MethyLight^{™} assay is combined with methylation specific blocking probes covering CpG positions between the amplification primers.

The term "annealing", when used with respect to an oligonucleotide, is to be understood as a bond of an oligonucleotide to an at least substantially complementary sequence along the lines of the Watson-Crick base pairings in the sample DNA, forming a duplex structure, under moderate or stringent hybridization conditions. When it is used with respect to a single nucleotide or base, it refers to the binding according to Watson-Crick base pairings, e.g. C-G, A-T and A-U. Stringent hybridization conditions involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderate conditions involve washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

"Substantially complementary" means that an oligonucleotide does not need to reflect the exact sequence of the template and can comprise mismatches and/or spacers as defined herein. "Substantially identical" means that an oligonucleotide does not need to be 100% identical to a reference sequence but can comprise mismatches and/or spacers as defined herein. It is preferred that a substantially complementary or identical oligonucleotide comprises 1 to 3, i.e. 1, 2 or 3 mismatches and/or spacers, preferably one mismatch or spacer per oligonucleotide, such that the intended annealing does not not fail due to the mismatches and/or spacers.. To enable annealing despite mismatches and/or spacers, it is preferred that an oligonucleotide does not comprise more than 1 mismatch per 10 nucleotides (rounded up if the first decimal is 5 or higher, otherwise rounded down) of the oligonucleotide.

The term "oligonucleotide" as used herein refers to a linear oligomer of 5 to 50 ribonucleotides or preferably deoxyribonucleotides. Preferably, it has the structure of a single-stranded DNA fragment.

The term "primer oligonucleotide" as used herein refers to a single-stranded oligonucleotide sequence substantially complementary to a nucleic acid sequence sought to be copied (the template) and serves as a starting point for synthesis of a primer extension product. Preferably, a primer oligonucleotide is 10 to 40 nucleotides, more preferably 15-30 nucleotides and most preferably 19 to 25 nucleotides in length.

The term "blocker" as used herein refers to a molecule which binds in a methylation-specific manner to a single-strand of DNA (i.e. it is specific for either the converted methylated or preferably for the converted unmethylated DNA or the amplified DNA derived from it) and prevents amplification of the DNA by binding to it, for example by preventing a primer to bind or by preventing primer extension where it binds. Non-limiting examples for blockers are sequence and/or methylation specific antibodies (blocking e.g. primer binding or the polymerase) and in particular blocker oligonucleotides.

A "blocker oligonucleotide" may be a blocker that prevents the extension of the primer located upstream of the blocker oligonucleotide. It comprises nucleosides/nucleotides having a backbone resistant to the 5' nuclease activity of the polymerase. This may be achieved, for example, by comprising peptide nucleic acid (PNA), locked nucleic acid (LNA), Morpholino, glycol nucleic acid (GNA), threose nucleic acid (TNA), bridged nucleic acids (BNA), N3'-P5' phosphoramidate (NP) oligomers, minor groove binder-linked-oligonucleotides (MGB- linked oligonucleotides), phosphorothioate (PS) oligomers, CrC₄alkylphosphonate oligomers, phosphoramidates, β-phosphodiester oligonucleotides, α-phosphodiester oligonucleotides or a combination thereof. Alternatively, it may be a non-extendable oligonucleotide with a binding site on the DNA single-strand that overlaps with the binding site of a primer oligonucleotide. When the blocker is bound, the primer cannot bind and therefore the amplicon is not generated. When the blocker is not bound, the primer-binding site is accessible and the amplicon is generated. For such an overlapping blocker, it is preferable that the affinity of the blocker is higher than the affinity of the primer for the DNA. A blocker oligonucleotide is typically 15 to 50, preferably 20 to 40 and more preferably 25 to 35 nucleotides long. "At least one blocker" refers in particular to a number of 1, 2, 3, 4 or 5 blockers, more particularly to 1-2 or 1-3 blockers. Also, a blocker oligonucleotide cannot by itself act as a primer (i.e. cannot be extended by a polymerase) due to a non-extensible 3' end.

The term "probe oligonucleotide" or "probe" as used herein refers to an oligonucleotide that is used to detect an amplicon by annealing to one strand of the amplicon, usually not where any of the primer oligonucleotides binds (i.e. not to a sequence segment of the one strand which overlaps with a sequence segment a primer oligonucleotide anneals to). Preferably it anneals without a mismatch or spacer, in other words it is preferably complementary to one strand of the amplicon. A probe oligonucleotide is preferably 5-40 nucleotides, more preferably 10 to 25 and most preferably 25 to 20 nucleotides long. Usually, the probe is linked, preferably covalently linked, to at least one detectable label which allows detection of the amplicon and/or at least one quencher which allows quenching the signal of a (preferably the) detectable label. The term "detectable label" as used herein does not exhibit any particular limitation. The detectable label may be selected from the group consisting of radioactive labels, luminescent labels, fluorescent dyes, compounds having an enzymatic activity, magnetic labels, antigens, and compounds having a high binding affinity for a detectable label. For example, fluorescent dyes linked to a probe may serve as a detection label, e.g. in a real-time PCR. Suitable radioactive markers are P-32, S-35, I-125, and H-3, suitable luminescent markers are chemiluminescent compounds, preferably luminol, and suitable fluorescent markers are preferably dansyl chloride, fluorcein-5-isothiocyanate, and 4-fluor-7-nitrobenz-2-aza-1,3 diazole, in particular 6-Carboxyfluorescein (FAM), 6-Hexachlorofluorescein (HEX), 5(6)-Carboxytetramethylrhodamine (TAMRA), 5(6)-Carboxy-X-Rhodamine (ROX), Cyanin-5-Fluorophor (Cy5) and derivates thereof; suitable enzyme markers are horseradish peroxidase, alkaline phosphatase, α-galactosidase, acetylcholinesterase, or biotin. A probe may also be linked to a quencher. The term "quencher" as used herein refers to a molecule that deactivates or modulates the signal of a corresponding detectable label, e.g. by energy transfer, electron transfer, or by a chemical mechanism as defined by IUPAC (see compendium of chemical terminology 2nd ed. 1997). In particular, the quencher modulates the light emission of a detectable label that is a fluorescent dye. In some cases, a quencher may itself be a fluorescent molecule that emits fluorescence at a characteristic wavelength distinct from the label whose fluorescence it is quenching. In other cases, the quencher does not itself fluoresce (i.e., the quencher is a "dark acceptor"). Such quenchers include, for example, dabcyl, methyl red, the QSY diarylrhodamine dyes, and the like.

The term "covering a CpG site" as used herein with respect to an oligonucleotide refers to the oligonucleotide annealing to a region of DNA comprising this CpG site, before or after conversion of the C of the CpG site (i.e. the CpG site of the corresponding genomic DNA when it is referred to a bisulfite converted sequence). The annealing may, with respect to the CpG site (or former CpG site if the C was converted), be methylation-specific or methylationunspecific as described below.

The term "methylation-specific" as used herein refers generally to the dependency from the presence or absence of CpG methylation.

The term "methylation-specific" as used herein with respect to an oligonucleotide means that the oligonucleotide does or does not anneal to a single-strand of DNA (in which cytosine unmethylated in the 5-position has been converted to uracil or another base that does not hybridize to guanine, and where it comprises at least one CpG site before conversion) without a mismatch regarding the position of the C in the at least one CpG site, depending on whether the C of the at least one CpG sites was unmethylated or methylated prior to the conversion, i.e. on whether the C has been converted or not. The methylation-specificity can be either positive (the oligonucleotide anneals without said mismatch if the C was not converted) or negative (the oligonucleotide anneals without said mismatch if the C was converted). To prevent annealing of the oligonucleotide contrary to its specificity, it preferably covers at least 2, 3, 4, 5 or 6 and preferably 3 to 6 CpG sites before conversion.

The term "methylation-unspecific" as used herein refers generally to the independency from the presence or absence of CpG methylation.

The term "methylation-unspecific" as used herein with respect to an oligonucleotide means that the oligonucleotide does anneal to a single-strand of DNA (in which cytosine unmethylated in the 5-position has been converted to uracil or another base that does not hybridize to guanine, and where it may or may not comprise at least one CpG site before conversion) irrespective of whether the C of the at least one CpG site was unmethylated or methylated prior to the conversion, i.e. of whether the C has been converted or not. In one case, the region of the single-strand of DNA the oligonucleotide anneals to does not comprise any CpG sites (before and after conversion) and the oligonuclotide is methylation-unspecific solely for this reason. While a methylation-unspecific oligonucleotide may cover one or more CpG dinucleotides, it does so with mismatches and/ or spacers. The term "mismatch" as used herein refers to base-pair mismatch in DNA, more specifically a base-pair that is unable to form normal base-pairing interactions (i.e., other than "A" with "T" or "U", or "G" with "C").

An oligonucleotide according to the third aspect, i.e. a probe, blocker or primer, may also cover an SNP site with an SNP-unspecific mismatch or with a spacer.

The term "SNP site" as used herein refers to the site of an "SNP", i.e. a single nucleotide polymorphism at a particular position in the, preferably human, genome that varies among a population of individuals. SNPs of the genomic DNA the present application refers to are known in the art and can be found in online databases such as dbSNP of NCBI (http://www.ncbi.nlm.nih.gov/snp).

The term "SNP-unspecific mismatch" as used herein refers to a mismatch that is due to a nucleotide substitution that does not substitute the nucleotide with one that corresponds to a nucleotide that is found at the same position in the genome of another individual of the same population.

The term "spacer" as used herein refers to a non-nucleotide spacer molecule, which increases, when joining two nucleotides, the distance between the two nucleotides to about the distance of one nucleotide (i.e. the distance the two nucleotides would be apart if they were joined by a third nucleotide). Non-limiting examples for spacers are Inosine, d-Uracil, halogenated bases, Amino-dT, C3, C12, Spacer 9, Spacer 18, and dSpacer)

The term "reflects" as used herein is to be understood to mean "is a result of" or "shows".

The phrase "method for detecting the presence or absence of cancer in a subject" as used herein refers to a determination whether the subject has cancer or not. As will be understood by persons skilled in the art, such assessment normally may not be correct for 100% of the subjects, although it preferably is correct. The term, however, requires that a correct indication can be made for a statistically significant part of the subjects. Whether a part is statistically significant can be determined easily by the person skilled in the art using several well known statistical evaluation tools, for example, determination of confidence intervals, determination of p values, Student's t-test, Mann-Whitney test, etc. Details are provided in Dowdy and Wearden, Statistics for Research, John Wiley & Sons, New York 1983. The preferred confidence intervals are at least 50%, at least 60%, at least 70%, at least 80%, at least 90%, at least 95%. The p values are preferably 0.05, 0.01, or 0.005.

The term "risk thereof" with respect to the method for detecting the presence or absence of cancer in a subject refers to the detection of an increased risk of developing the cancer or an increased probability of having it. If the subject already has an increased risk in view of one or more risk factors that can be attributed to it (as defined herein), the 'risk therof refers to a risk that is increased further, i.e. that is in addition to the risk due to those risk factors.

The term "cancer" as used herein refers to a large family of diseases which involve abnormal cell growth with the potential to invade or spread to other parts of the body. The cells form a subset of neoplasms or tumors. A neoplasm or tumor is a group of cells that have undergone unregulated growth, and will often form a mass or lump, but may be distributed diffusely. Preferably, the term "cancer" is defined by one or more of the following characteristics:
- self-sufficiency in growth signalling,
- insensitivity to anti-growth signals,
- evasion of apoptosis,
- enabling of a limitless replicative potential,
- induction and sustainment of angiogenesis, and/or
- activation of metastasis and invasion of tissue.

The cancer with respect to the present invention is preferably selected from the group consisting of liver cancer, esophagus cancer, stomach cancer, pancreas cancer, breast cancer, lung cancer, neck cancer, kidney cancer, thyroid cancer, prostate cancer, bladder cancer, colon cancer, and ovary cancer.

The term "stomach cancer" or "gastric cancer" is used in the broadest sense and refers to all cancers that start in the stomach. It includes the subtypes adenocarcinoma, lymphoma, gastrointestinal stromal tumor (GIST), carcinoid tumor and aquamous cell carcinoma, small cell carcinoma and leiomyosarcoma starting in the stomach. It also includes the following stages (as defined by the corresponding TNM classification(s) in brackets): stage 0 (Tis, N0, M0), stage IA (T1, N0, M0), stage IB (T1, N1, M0; or T2, N0, M0), stage IIA (T1, N2, M0; T2, N1, M0; or T2, N0, M0), stage IIB (T1, N3, M0; T2, N2, M0; T3, N1, M0; or T4a, N0, M0), stage IIIA (T1, N2, M0; T2, N1, M0; or T2, N0, M0), stage IIIB (T3, N3, M0; T4a, N2, M0; or T4b, N0 or N1, M0), stage IIIC (T4a, N3, M0; or T4b, N2 or N3, M0), and stage IV (any other T, N, and M).

The term "liver cancer" or "hepatic cancer" is used in the broadest sense and refers to all cancers that start in the liver. It includes the subtypes hepatocellular carcinoma (HCC), cholangiocarcinoma (bile duct cancer), angiosarcoma and hepatoblastoma. It also includes the following stages (as defined by the corresponding TNM classification(s) in brackets): stage I (T1, N0, M0), stage II (T2, N0, M0), stage IIIA (T3a, N0, M0), stage IIIb (T3b, N0, M0), stage IIIc (T4, N0, M0), stage IVA (any T, N1, M0), stage IVB (any T, any N and M1).

The term "esophagus cancer" or "oesophageal cancer" is used in the broadest sense and refers to all cancers that start in the esophagus. It includes the subtypes esophageal squamous-cell carcinoma and esophageal adenocarcinoma. It also includes the following stages (as defined by the corresponding TNM classification(s) in brackets): For squamous cell carcinoma: stage 0 (Tis, N0, M0, GX or G1), stage IA (T1, N0, M0, GX or G1), stage IB (T1, N0, M0, G2 or G3; or T2 or T3, N0, M0, GX or G1), stage IIA (T2 or T3, N0, M0, GX or G1, location upper or middle; or T2 or T3, N0, M0, G2 or G3, location lower), stage IIB (T2 or T3, N0, M0, G2 or G3, location upper or middle; or T1 or T2, N1, M0), stage IIIA (T1 or T2, N2, M0; T3, N1, M0; or T4a, N0, M0), stage IIIC (T4a, N1 or N2, M0; T4b, any N, M0; or any T, N3, M0) and stage IV (any T, any N, M1); For adenocarcinoma: stage 0 (Tis, N0, M0, GX or G1), stage IA (T1, N0, M0, GX, G1 or G2), stage IB (T1, N0, M0, G3; or T2, N0, M0, GX, G1, or G2), stage IIA (T2, N0, M0, G3), stage IIB (T3, N0, M0; or T1 or T2, N1, M0), stage IIIA (T1 or T2, N2, M0; T3, N1, M0; or T3, N2, M0), stage IIIC (T4a, N1 or N2, M0; T4b, any N, M0; or any T, N3, M0), and stage IV (any T, any N, M1).

The term "pancreas cancer" is used in the broadest sense and refers to all cancers that start in the pancreas. It includes the subtypes exocrine cancers, endocrine cancers, pancreatoblastoma, sarcomas of the pancreas, and lymphoma. Exocrine cancers include adenocarcinomas, in particular ductal adenocarcinomas, as well as cystic tumours and cancer of the acinar cells. Endocrine cancers include gastrinomas, insulinomas, somatostatinomas, VIPomas, and glucagonomas. It also includes the following stages (as defined by the corresponding TNM classification(s) in brackets): stage 0 (Tis, N0, M0), stage IA (T1, N0, M0), stage IB (T2, N0, M0), stage IIA (T3, N0, M0), stage IIB (T1-3, N1, M0), stage III (T4, any N, M0), and stage IV (any T, any N, M1).

The term "breast cancer" is used in the broadest sense and refers to all cancers that start in the breast. It includes the subtypes ductal carcinoma in situ, invasive ductal carcinoma (including tubular carcinoma of the breast, medullary carcinoma of the breast, mucinous carcinoma of the breast, papillary carcinoma of the breast, and cribriform carcinoma of the breast), invasive lobular carcinoma, inflammatory breast cancer, lobular carcinoma in situ, male breast cancer, Paget's disease of the nipple, and Phyllodes tumors of the breast. It also includes the following stages (as defined by the corresponding TNM classification(s) in brackets): stage 0: (Tis, N0, M0), stage IA (T1, N0, M0), stage IB (T0 or T1, N1mi, M0), stage IIA (T0 or T1, N1 (but not N1mi), M0; or T2, N0, M0), stage IIB (T2, N1, M0; or T3, N0, M0), stage IIIA (T0 to T2, N2, M0; or T3, N1 or N2, M0), stage IIIB (T4, N0 to N2, M0), stage IIIC (any T, N3, M0), and stage IV (any T, any N, M1).

The term "neck cancer" or "head and neck cancer" is used in the broadest sense and refers to all cancers that start in the neck or in the head. It includes the subtypes laryngeal cancer, hypopharyngeal cancer, nasal cavity and paranasal sinus cancer, nasopharyngeal cancer, salivary gland cancer and oral and oropharyngeal cancer. It also includes the following stages (as defined by the corresponding TNM classification(s) in brackets): stage 0 (Tis, N0, M0), stage I (T1, N0, M0), stage II (T2, N0, M0), stage III (T3, N0, M0; or T1 to T3, N1, M0), stage IVA (T4a, N0 or N1, M0; or T1 to T4a, N2, M0), stage IVB (T4b, any N, M0 or any T, N3, M0), and stage IVC (any T, any N, M1).

The term "kidney cancer" or "renal cancer" is used in the broadest sense and refers to all cancers that start in the kidney. It includes the subtypes renal cell carcinoma or RCC (including clear cell RCC, papillary RCC, chromophobe RCC, and collecting duct RCC), transitional cell carcinoma, Wilms tumor, and renal sarcoma. It also includes the following stages (as defined by the corresponding TNM classification(s) in brackets): stage I (T1, N0, M0), stage II (T2, N0, M0), stage III (T3, N0, M0; or T1 to T3, N1, M0), and stage IV (T4, any N, M0; or any T, any N, M1).

The term "thyroid cancer" is used in the broadest sense and refers to all cancers that start in the thyroid. It includes the subtypes differentiated thyroid cancers (including papillary carcinoma, follicular carcinoma, and Hürthle cell carcinoma), medullary thyroid carcinoma, anaplastic carcinoma, thyroid lymphoma, and thyroid sarcoma. It also includes the following stages (as defined by the corresponding TNM classification(s) in brackets): For papillary or follicular (differentiated) thyroid cancer in patients younger than 45: stage I (any T, any N, M0), and stage II (any T, any N, M1); for papillary or follicular (differentiated) thyroid cancer in patients 45 years and older: stage I (T1, N0, M0), stage II (T2, N0, M0), stage III (T3, N0, M0; or T1 to T3, N1a, M0), stage IVA (T4a, any N, M0; or T1 to T3, N1b, M0), stage IVB (T4b, any N, M0), and stage IVC (any T, any N, M1); for medullary thyroid cancer: stage I (T1, N0, M0), stage II (T2, N0, M0; or T3, N0, M0), stage III (T1 to T3, N1a, M0), stage IVA (T4a, any N, M0; or T1 to T3, N1b, M0), stage IVB (T4b, any N, M0), and stage IVC (any T, any N, M1); for anaplastic (undifferentiated) thyroid cancer: stage IVA (T4a, any N, M0), stage IVB (T4b, any N, M0), and stage IVC (any T, any N, M1).

The term "prostate cancer" is used in the broadest sense and refers to all cancers that start in the prostate. It includes the subtypes adenocarcinoma, sarcoma, small cell carcinoma, neuroendocrine tumor (other than small cell carcinoma), and transitional cell carcinomas. It also includes the following stages (as defined by the corresponding TNM classification(s) in brackets): stage I (T1, N0, M0, Gleason score 6 or less, PSA less than 10; or T2a, N0, M0, Gleason score 6 or less, PSA less than 10), stage IIA (T1, N0, M0, Gleason score of 7, PSA less than 20; T1, N0, M0, Gleason score of 6 or less, PSA at least 10 but less than 20; or T2a or T2b, N0, M0, Gleason score of 7 or less, PSA less than 20), stage IIB (T2c, N0, M0, any Gleason score, any PSA; T1 or T2, N0, M0, any Gleason score, PSA of 20 or more; or T1 or T2, N0, M0, Gleason score of 8 or higher, any PSA), stage III (T3, N0, M0, any Gleason score, any PSA) and stage IV (T4, N0, M0, any Gleason score, any PSA; any T, N1, M0, any Gleason score, any PSA; or any T, any N, M1, any Gleason score, any PSA).

The term "bladder cancer" is used in the broadest sense and refers to all cancers that start in the bladder. It includes the subtypes transitional cell (urothelial) carcinoma (including papillary and flat carcinomas), squamous cell carcinoma, adenocarcinoma, small cell carcinoma, and sarcoma. It also includes the following stages (as defined by the corresponding TNM classification(s) in brackets): stage 0a (Ta, N0, M0), stage 0is (Tis, N0, M0), stage I (T1, N0, M0), stage II (T2a or T2b, N0, M0), stage III (T3a, T3b, or T4a, N0, M0), and stage IV (T4b, N0, M0; any T, N1 to N3, M0; or any T, any N, M1).

The term "ovary cancer" or "ovarian cancer" is used in the broadest sense and refers to all cancers that start in the ovaries. It includes the subtypes benign epithelial ovarian tumors, tumors of low malignant potential, and malignant epithelial ovarian tumors. It also includes the following stages (as defined by the corresponding TNM classification(s) in brackets): stage IA (T1a, N0, M0), stage IB (T1b, N0, M0), stage IC (T1c, N0, M0), stage IIA (T2a, N0, M0), stage IIB (T2b, N0, M0), stage IIIA1 (T1 or T2, N1, M0), stage IIIA2 (T3 a2, N0 or N1, M0), stage IIIB (T3b, N0 or N1, M0), stage IIIC (T3c, N0 or N1, M0), and stage IV (any T, any N, M1)

The term "colon cancer" or "colorectal cancer" is used in the broadest sense and refers to (1) all stages and all forms of cancer arising from epithelial cells of the large intestine and/or rectum and/or (2) all stages and all forms of cancer affecting the lining of the large intestine and/or rectum. It includes the subtypes adenocarcinoma, gastrointestinal carcinoid tumor, gastrointestinal stromal tumor, primary colorectal lymphoma, leiomyosarcoma, melanoma or squamous cell carcinoma, each originating from the colon (colon cancer) or the rectum (rectal cancer). In the staging systems used for classification of colorectal cancer, the colon and rectum are treated as one organ. It also includes the following stages (as defined by the corresponding TNM classification(s) in brackets): stage 0 (Tis, N0, M0), stage I (T1-T2, N0, M0), stage IIA (T3, N0, M0), stage IIB (T4a, N0, M0), stage IIC (T4b, N0, M0), stage IIIA (T1-T2, N1, M0; or T1, N2a, M0), stage IIIB (T3-T4a, N1, M0; T2-T3, N2a, M0; or T1-T2, N2b, M0), stage IIIC (T4a, N2a, M0; T3-T4a, N2b, M0; or T4b N1-N2 M0), and stage IVA (any T, any N, M1a), stage IVA (any T, any N, M1b).

The term "lung cancer" is used in the broadest sense and refers to all cancers that start in the lung. It includes the subtypes small cell lung cancer (SCLC) and non-small cell lung cancer (NSCLC). The term "SCLC" or "small cell lung cancer", as used herein, refers to an undifferentiated neoplasm, preferably composed of primitive- or embryonic-appearing cells. As the name implies, the cells in small-cell carcinomas are smaller than normal cells and barely have room for any cytoplasm. The term "NSCLC" or "non-small cell lung cancer", as used herein, refers to a group of heterogeneous diseases grouped together because their prognosis and management is roughly identical and includes, according to the histological classification of the World Health Organization/International Association for the Study of Lung Cancer (Travis WD et al. Histological typing of lung and pleural tumors. 3rd ed. Berlin: Springer-Verlag, 1999):
(i) Squamous cell carcinoma (SCC), accounting for 30% to 40% of NSCLC, starts in the larger breathing tubes but grows slower meaning that the size of these tumors varies on diagnosis.
(ii) Adenocarcinoma is the most common subtype of NSCLC, accounting for 50% to 60% of NSCLC, which starts near the gas-exchanging surface of the lung and which includes a subtype, the bronchioalveolar carcinoma, which may have different responses to treatment.
(iii) Large cell carcinoma is a fast-growing form that grows near the surface of the lung. It is primarily a diagnosis of exclusion, and when more investigation is done, it is usually reclassified to squamous cell carcinoma or adenocarcinoma.
(iv) Adenosquamous carcinoma is a type of cancer that contains two types of cells: squamous cells (thin, flat cells that line certain organs) and gland-like cells.
(v) Carcinomas with pleomorphic, sarcomatoid or sarcomatous elements. This is a group of rare tumors reflecting a continuum in histological heterogeneity as well as epithelial and mesenchymal differentiation.
(vi) Carcinoid tumor is a slow-growing neuroendocrine lung tumor and begins in cells that are capable of releasing a hormone in response to a stimulus provided by the nervous system.
(vii) Carcinomas of salivary gland type begin in salivary gland cells located inside the large airways of the lung.
(viii) Unclassified carcinomas include cancers that do not fit into any of the aforementioned lung cancer categories.

The NSCLC may be a squamous cell carcinoma, adenocarcinoma, large cell (undifferentiated) carcinoma, adenosquamous carcinoma and sarcomatoid carcinoma.

The lung cancer may be a stage 0, IA, IB, IIa, IIb, IIIa, IIIb or IV lung cancer as defined by the corresponding TNM classification(s) in brackets: stage 0 (TisN0M0), stage IA (T1, N0, M0), stage IB (T2, M0, N0), stage IIA (T1, N1, M0; or T2, N1, M0), stage IIB (T3, N0, M0), stage IIIA (T1, N2 M0; T2, N2, M0; T3, N1, M0; T3, N2, M0; T4, N0, M0; or T4, N1, M0), stage IIIB (T1, N3, M0; T2, N3, M0; T3, N3, M0; T4, N2, M0; or T4, N3, M0), and stage IV (any T or any N with M1).

The TNM classification is a staging system for malignant cancer. As used herein the term "TNM classification" refers to the 6^{th} edition of the TNM stage grouping as defined in Sobin et al. (International Union Against Cancer (UICC), TNM Classification of Malignant tumors, 6th ed. New York; Springer, 2002, pp. 191-203).

The term "cancer cell" as used herein refers to a cell that acquires a characteristic set of functional capabilities during their development, particularly one or more of the following: the ability to evade apoptosis, self-sufficiency in growth signals, insensitivity to anti-growth signals, tissue invasion/metastasis, significant growth potential, and/or sustained angiogenesis. The term is meant to encompass both pre-malignant and malignant cancer cells.

The term "tumor DNA" or "tumor DNA of a cancer cell" as used herein refers simply to DNA of a cancer cell. It is used only to distinguish DNA of a cancer cell more clearly from other DNA referred to herein. Thus, unless ambiguities are introduced, the term "DNA of a cancer cell" may be used instead.

The term "subject" as used herein refers to an individual, such as a human, a non-human primate (e.g. chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term does not denote a particular age or sex. In a particular meaning, the subject is a mammal. In a preferred meaning, the subject is a human. In principle, the subject can be any subject of which the methylation status within genomic DNA having a sequence comprised in SEQ ID NO: 1 and/or within genomic DNA having a sequence comprised in SEQ ID NO: 26, in particular from a sample comprising cell-free DNA from blood or a sample derived therefrom of a subject, is not known. Depending on what the method of the first aspect is to be used for, the term "subject" may have different limitations. For example, it the method is to be used for detecting cancer or screening subjects for cancer, the subject is not known to have cancer, i.e. it may or may not have cancer. In this example, the subject preferably is at risk or increased risk or is suspected to have cancer. "At risk or increased risk" means that one or more risk factors can be attributed to the subject), preferably as defined by the American Cancer Society generally or for the respective cancer.

The term "is indicative for" or "indicates" as used herein refers to an act of identifying or specifying the thing to be indicated. As will be understood by persons skilled in the art, such assessment normally may not be correct for 100% of the subjects, although it preferably is correct. The term, however, requires that a correct indication can be made for a statistically significant part of the subjects. For a description of statistic significance and suitable confidence intervals and p values, see above.

The term "amplifying" or "generating an amplicon" as used herein refers to amplifying a defined region of a double-stranded or single-stranded DNA template, typically with a polymerase chain reaction (PCR). An "amplicon" is a double-stranded fragment of DNA according to said defined region.

The term "pair of primers" as used herein refers to two oligonucleotides, namely a forward and a reverse primer, that have, with respect to a double-stranded nucleic acid molecule, sequences that are (at least substantially) identical to one strand each such that they each anneal to the complementary strand of the strand they are (at least substantially) identical to. The term "forward primer" refers to the primer which is (at least substantially) identical to the forward strand (as defined by the direction of the genomic reference sequence) of the double-stranded nucleic acid molecule, and the term "reverse primer" refers to the primer which is (at least substantially) identical to the reverse complementary strand of the forward strand in the double-stranded nucleic acid molecule. The distance between the sites where forward and reverse primer anneal to their template depends on the length of the amplicon the primers are supposed to allow generating. Typically, with respect to the present invention it is between 40 and 1000 bp. Preferred amplicon sizes are specified herein. In case of single-stranded DNA template that is to be amplified using a pair of primers, only one of the primers anneals to the single strand in the first amplification cycle. The other primer then binds to the newly generated complementary strand such that the result of amplification is a double-stranded DNA fragment. The phrase "pair of primers suitable for generating an amplicon from a single strand of genomic DNA in which cytosine unmethylated in the 5-position has been converted to uracil or another base that does not hybridize to guanine" refers to a pair of primers which takes into account a base change from unmethylated cytosines to uracil, which basepairs with adenine and is therefore replaced with thymine in the amplicon.

The term "diagnosis" as used herein refers to a determination whether a subject does or does not have cancer, and preferably also which cancer. A diagnosis by methylation analysis of the target DNA as described herein may be supplemented with a further means as described herein to and/or narrow down the cancer detected with the methylation analysis. As will be understood by persons skilled in the art, the diagnosis normally may not be correct for 100% of the subjects, although it preferably is correct. The term, however, requires that a correct diagnosis can be made for a statistically significant part of the subjects. For a description of statistic significance and suitable confidence intervals and p values, see above.

The term "prognosis", as used herein, means the likelihood of recovery from a disease or the prediction of the probable development or outcome of a disease, including but not limited to predicting the length of overall survival (OS), 1 year survival (1YS), response to therapy (RT) disease-free survival, progression-free survival, and event-free survival. Depending on whether the probable development or outcome is positive or negative, the prognosis is good or bad, respectively. As will be understood by persons skilled in the art, the prognosis normally may not be correct for 100% of the subjects, although it preferably is correct. The term, however, requires that a correct prognosis can be made for a statistically significant part of the subjects. For a description of statistic significance and suitable confidence intervals and p values, see above.

The term "predicting the effect of a cancer treatment" as used herein refers to the expected outcome of the cancer disease in response to the treatment and relates to the assessment of its state of development, progression, or of its regression, and/or the prognosis of the course of the cancer in the future. In particular, it is envisaged that the presence or absence of DNA methylation is detected as described herein prior to the cancer treatment and immediately after the start of the treatment, wherein immediately means within 0.5 to 7 days, preferably within 1, 2 or 3 days. An increase in methylated DNA in the sample immediately after the start of the treatment predicts that the cancer treatment has a therapeutic effect. It is thought that an effective treatment leads to an increase of cell-free DNA in the sample for example due to a destruction of cancer cells. As will be understood by persons skilled in the art, the prediction normally may not be correct for 100% of the subjects, although it preferably is correct. The term, however, requires that a correct prediction can be made for a statistically significant part of the subjects. For a description of statistic significance and suitable confidence intervals and p values, see above.

The term "classification" as used herein refers to refers to assigning the cancer or subject into a group. Preferably, the group can be one according to the other methods of the fifth aspect, e.g. 'subject diagnosed with cancer', 'subject with a good/bad prognosis', 'subject with a predicted effect or without predicted effect of a cancer treatment', 'subject responding or not responding to a cancer treatment', or 'subject with increased likelihood of cancer'. Also, the classifications can simply be 'subject with DNA methylation in genomic DNA according to the respective SEQ ID NO'. As will be understood by persons skilled in the art, the classification normally may not be correct for 100% of the subjects, although it preferably is correct. The term, however, requires that a correct classification can be made for a statistically significant part of the subjects. For a description of statistic significance and suitable confidence intervals and p values, see above.

The phrase "response to treatment", "response to therapy" as used herein refers to the response of a subject suffering from cancer to a therapy for treating said disease. Standard criteria (Miller, et al., Cancer, 1981; 47(1): 207-14) can be used herewith to evaluate the response to therapy include response, stabilization and progression. The term "response", as used herein, can be a complete response (or complete remission) which is the disappearance of all detectable malignant disease or a partial response which is defined as approximately >50% decrease in the sum of products of the largest perpendicular diameters of one or more lesions (tumor lesions), no new lesions and no progression of any lesion. Subjects achieving complete or partial response are considered "responders", and all other subjects are considered "non-responders". The term "stabilization", as used herein, is defined as a < 50% decrease or a < 25% increase in tumor size. The term "progression", as used herein, is defined as an increase in the size of tumor lesions by > 25% or appearance of new lesions.

The term "monitoring" as used herein refers to the accompaniment of a diagnosed cancer during a treatment procedure or during a certain period of time, typically during at least 1 week, 2 weeks, 3 weeks, 4 weeks, 1 month, 2 months, 3 months, 4 months, 5 months, 6 months, 1 year, 2 years, 3 years, 5 years, 10 years, or any other period of time. The term "accompaniment" means that states of and, in particular, changes of these states of a cancer may be detected based on the amount of methylated target DNA, particular based on changes in the amount in any type of periodical time segment, determined e.g., daily or 1, 2, 3, 4, 5, 6, 7, 8, 9, 10, 11, 12, 13, 14 or 15 times per month (no more than one determination per day) over the course of the treatment, which may be up to 1, 2, 3, 4, 5, 6, 7, 8, 9, 10, 11, 12, 15 or 24 months. Amounts or changes in the amounts can also be determined at treatment specific events, e.g. before and/or after every treatment cycle or drug/therapy administration. A cycle is the time between one round of treatment until the start of the next round. Cancer treatment is usually not a single treatment, but a course of treatments. A course usually takes between 3 to 6 months, but can be more or less than that. During a course of treatment, there are usually between 4 to 8 cycles of treatment. Usually a cycle of treatment includes a treatment break to allow the body to recover. As will be understood by persons skilled in the art, the result of the monitoring normally may not be correct for 100% of the subjects, although it preferably is correct. The term, however, requires that a correct result of the monitoring can be achieved for a statistically significant part of the subjects. For a description of statistic significance and suitable confidence intervals and p values, see above.

The phrase "screening of subjects to detect an increased likelihood of cancer" refers to the use of the method of the first aspect with samples of a population of subjects. Preferably, the subjects have an increased risk for or are suspected of having a cancer selected from the groups recited herein with respect to certain SEQ ID NOs. In particular, one or more of the following risk factors recited herein can be attributed to the subjects of the population. In a specific embodiment, the same one or more risk factors can be attributed to all subjects of the population. For example, the population may be characterized by a certain minimal age (e.g. 50 or older). It is to be understood that the term "screening" does not necessarily indicate a definite diagnosis, but is intended to indicate an increased possibility of the presence or of the absence of a certain cancer or of a set of cancers. An indicated increased possibility is preferably confirmed and/or narrowed down using a further means as described herein. As will be understood by persons skilled in the art, the screening result normally may not be correct for 100% of the subjects, although it preferably is correct. The term, however, requires that a correct screening result can be achieved for a statistically significant part of the subjects. For a description of statistic significance and suitable confidence intervals and p values, see above.

The term "treatment" or "treating" with respect to cancer as used herein refers to a therapeutic treatment, wherein the goal is to reduce progression of cancer. Beneficial or desired clinical results include, but are not limited to, release of symptoms, reduction of the length of the disease, stabilized pathological state (specifically not deteriorated), slowing down of the disease's progression, improving the pathological state and/or remission (both partial and total), preferably detectable. A successful treatment does not necessarily mean cure, but it can also mean a prolonged survival, compared to the expected survival if the treatment is not applied. In a preferred embodiment, the treatment is a first line treatment, i.e. the cancer was not treated previously. Cancer treatment involves a treatment regimen.

The term "treatment regimen" as used herein refers to how the subject is treated in view of the disease and available procedures and medication. Non-limiting examples of cancer treatment regimes are chemotherapy, surgery and/or irradiation or combinations thereof. The early detection of cancer the present invention enables allows in particular for a surgical treatment, especially for a curative resection. In particular, the term "treatment regimen" refers to administering one or more anti-cancer agents or therapies as defined below. The term "anti-cancer agent or therapy" as used herein refers to chemical, physical or biological agents or therapies, or surgery, including combinations thereof, with antiproliferative, antioncogenic and/or carcinostatic properties.

A chemical anti-cancer agent or therapy may be selected from the group consisting of alkylating agents, antimetabolites, plant alkaloyds and terpenoids and topoisomerase inhibitors. Preferably, the alykylating agents are platinum-based compounds. In one embodiment, the platinum-based compounds are selected from the group consisting of cisplatin, oxaliplatin, eptaplatin, lobaplatin, nedaplatin, carboplatin, iproplatin, tetraplatin, lobaplatin, DCP, PLD-147, JMl 18, JM216, JM335, and satraplatin.

A physical anti-cancer agent or therapy may be selected from the group consisting of radiation therapy (e.g. curative radiotherapy, adjuvant radiotherapy, palliative radiotherapy, teleradiotherapy, brachytherapy or metabolic radiotherapy), phototherapy (using, e.g. hematoporphoryn or photofrin II), and hyperthermia.

Surgery may be a curative resection, palliative surgery, preventive surgery or cytoreductive surgery. Typically, it involves an excision, e.g. intracapsular excision, marginal, extensive excision or radical excision as described in Baron and Valin (Rec. Med. Vet, Special Canc. 1990; 11(166):999-1007).

A biological anti-cancer agent or therapy may be selected from the group consisting of antibodies (e.g. antibodies stimulating an immune response destroying cancer cells such as retuximab or alemtuzubab, antibodies stimulating an immune response by binding to receptors of immune cells an inhibiting signals that prevent the immune cell to attack "own" cells, such as ipilimumab, antibodies interfering with the action of proteins necessary for tumor growth such as bevacizumab, cetuximab or panitumumab, or antibodies conjugated to a drug, preferably a cell-killing substance like a toxin, chemotherapeutic or radioactive molecule, such as Y-ibritumomab tiuxetan, I-tositumomab or ado-trastuzumab emtansine), cytokines (e.g. interferons or interleukins such as INF-alpha and IL-2), vaccines (e.g. vaccines comprising cancer-associated antigens, such as sipuleucel-T), oncolytic viruses (e.g. naturally oncolytic viruses such as reovirus, Newcastle disease virus or mumps virus, or viruses genetically engineered viruses such as measles virus, adenovirus, vaccinia virus or herpes virus preferentially targeting cells carrying cancer-associated antigens such as EGFR or HER-2), gene therapy agents (e.g. DNA or RNA replacing an altered tumor suppressor, blocking the expression of an oncogene, improving a subject's immune system, making cancer cells more sensitive to chemotherapy, radiotherapy or other treatments, inducing cellular suicide or conferring an anti-angiogenic effect) and adoptive T cells (e.g. subject-harvested tumor-invading T-cells selected for antitumor activity, or subject-harvested T-cells genetically modified to recognize a cancer-associated antigen) .

In one embodiment, the one or more anti-cancer drugs is/are selected from the group consisting of Abiraterone Acetate, ABVD, ABVE, ABVE-PC, AC, AC-T, ADE, Ado-Trastuzumab Emtansine, Afatinib Dimaleate, Aldesleukin, Alemtuzumab, Aminolevulinic Acid, Anastrozole, Aprepitant, Arsenic Trioxide, Asparaginase Erwinia chrysanthemi, Axitinib, Azacitidine, BEACOPP, Belinostat, Bendamustine Hydrochloride, BEP, Bevacizumab, Bexarotene, Bicalutamide, Bleomycin, Bortezomib, Bosutinib, Brentuximab Vedotin, Busulfan, Cabazitaxel, Cabozantinib-S-Malate, CAFCapecitabine, CAPOX, Carboplatin, CARBOPLATIN-TAXOL, Carfilzomib, Carmustine, Carmustine Implant, Ceritinib, Cetuximab, Chlorambucil, CHLORAMBUCIL-PREDNISONE, CHOP, Cisplatin, Clofarabine, CMF, COPP, COPP-ABV, Crizotinib, CVP, Cyclophosphamide, Cytarabine, Cytarabine, Liposomal, Dabrafenib, Dacarbazine, Dactinomycin, Dasatinib, Daunorubicin Hydrochloride, Decitabine, Degarelix, Denileukin Diftitox, Denosumab, Dexrazoxane Hydrochloride, Docetaxel, Doxorubicin Hydrochloride, Doxorubicin Hydrochloride Liposome, Eltrombopag Olamine, Enzalutamide, Epirubicin Hydrochloride, EPOCH, Eribulin Mesylate, Erlotinib Hydrochloride, Etoposide Phosphate, Everolimus, Exemestane, FEC, Filgrastim, Fludarabine Phosphate, Fluorouracil, FU-LV, Fulvestrant, Gefitinib, Gemcitabine Hydrochloride, GEMCITABINE-CISPLATIN, GEMCITABINE-OXALIPLATIN, Gemtuzumab Ozogamicin, Glucarpidase, Goserelin Acetate, HPV Bivalent Vaccine, Recombinant HPV Quadrivalent Vaccine, Hyper-CVAD, Ibritumomab Tiuxetan, Ibrutinib, ICE, Idelalisib, Ifosfamide, Imatinib, Mesylate, Imiquimod, Iodine 131 Tositumomab and Tositumomab, Ipilimumab, Irinotecan Hydrochloride, Ixabepilone, Lapatinib Ditosylate, Lenalidomide, Letrozole, Leucovorin Calcium, Leuprolide Acetate, Liposomal Cytarabine, Lomustine, Mechlorethamine Hydrochloride, Megestrol Acetate, Mercaptopurine, Mesna, Methotrexate, Mitomycin C, Mitoxantrone Hydrochloride, MOPP, Nelarabine, Nilotinib, Obinutuzumab, Ofatumumab, Omacetaxine Mepesuccinate, OEPA, OFF, OPPA, Oxaliplatin, Paclitaxel, Paclitaxel Albumin-stabilized Nanoparticle Formulation, PAD, Palifermin, Palonosetron Hydrochloride, Pamidronate Disodium, Panitumumab, Pazopanib Hydrochloride, Pegaspargase, Peginterferon Alfa-2b, Pembrolizumab, Pemetrexed Disodium, Pertuzumab, Plerixafor, Pomalidomide, Ponatinib Hydrochloride, Pralatrexate, Prednisone, Procarbazine Hydrochloride, Radium 223 Dichloride, Raloxifene Hydrochloride, Ramucirumab, Rasburicase, R-CHOP, R-CVP, Recombinant HPV Bivalent Vaccine, Recombinant HPV Quadrivalent Vaccine, Recombinant Interferon Alfa-2b, Regorafenib, Rituximab, Romidepsin, Romiplostim, Ruxolitinib Phosphate, Siltuximab, Sipuleucel-T, Sorafenib Tosylate, STANFORD V, Sunitinib Malate, TAC, Talc, Tamoxifen Citrate, Temozolomide, Temsirolimus, Thalidomide, Topotecan Hydrochloride, Toremifene, Tositumomab and I 131 Iodine Tositumomab, TPF, Trametinib, Trastuzumab, Vandetanib, VAMP, VeIP, Vemurafenib, Vinblastine Sulfate, Vincristine Sulfate, Vincristine Sulfate Liposome, Vinorelbine Tartrate, Vismodegib, Vorinostat, XELOX, Ziv-Aflibercept, and Zoledronic Acid.

### SEQ IDs referred to in the application

The present application refers to SEQ ID NOs 1-63. An overview and explanation of these SED IDs is given in the following Table 1:

**Table 1: SEQ IDs of the specification. rc means reverse complement, C to T or G to A means converted by bisulfite conversion of cytosines outside of CpG context into uracil and replaced by thymidine in subsequent amplification. bis1 refers to the bisulfite converted forward strand (as recited in the SEQ ID of the respective genomic DNA) and bis2 to the bisulfite converted reverse complement strand of the forward strand (reverse complement of the SEQ ID of the respective genomic DNA), whereby the direction of the strand is defined by the direction of the genomic reference sequence as e.g. obtained from the genome build (HCGR38). For a mapping of the sequences, see Figure 1.**

| | | | |
|---|---|---|---|
| ANKRD13B gene associated region 17: 29584769-29619761 | | FOXF2 gene 6:1389834-1395597 | |
| SEQ ID NO: 1 | genomic reference | SEQ ID NO: 31 | genomic reference |
| SEQ ID NO: 2 | C to T (bis1) | SEQ ID NO: 32 | C to T (bis1) |
| SEQ ID NO: 3 | rc C to T (bis1) | SEQ ID NO: 33 | rc C to T (bis1) |
| SEQ ID NO: 4 | G to A (bis2 rc) | SEQ ID NO: 34 | G to A (bis2 rc) |
| SEQ ID NO: 5 | G to A (bis2 rc) rc | SEQ ID NO: 35 | G to A (bis2 rc) rc |
| | | | |
| ANKRD13B gene 17: 29589769-29614761 | | FOXF2 assay + CpG island1 6:1388905- | |
| SEQ ID NO: 6 | genomic reference | 1391158 | |
| SEQ ID NO: 7 | C to T (bis1) | SEQ ID NO: 36 | genomic reference |
| SEQ ID NO: 8 | rc C to T (bis1) | SEQ ID NO: 37 | C to T (bis1) |
| SEQ ID NO: 9 | G to A (bis2 rc) | SEQ ID NO: 38 | rc C to T (bis1) |
| SEQ ID NO: 10 | G to A (bis2 rc) rc | SEQ ID NO: 39 | G to A (bis2 rc) |
| | | SEQ ID NO: 40 | G to A (bis2 rc) rc |
| | | | |
| ANKRD13B assay+CpG island 17: 29612281-29613752 | | FOXF2 extended assay 6:1389866-1390941 | |
| SEQ ID NO: 11 | genomic reference | SEQ ID NO: 41 | genomic reference |
| SEQ ID NO: 12 | C to T (bis1) | SEQ ID NO: 42 | C to T (bis1) |
| SEQ ID NO: 13 | rc C to T (bis1) | SEQ ID NO: 43 | rc C to T (bis1) |
| SEQ ID NO: 14 | G to A (bis2 rc) | SEQ ID NO: 44 | G to A (bis2 rc) |
| SEQ ID NO: 15 | G to A (bis2 rc) rc | SEQ ID NO: 45 | G to A (bis2 rc) rc |
| | | | |
| ANKRD13B extended assay 17: 29613051-29614143 | | FOXF2 assay + MicroRNA MIR6720 6: 1390314-1390441 | |
| SEQ ID NO: 16 | genomic reference | SEQ ID NO: 46 | genomic reference |
| SEQ ID NO: 17 | C to T (bis1) | SEQ ID NO: 47 | C to T (bis1) |
| SEQ ID NO: 18 | rc C to T (bis1) | SEQ ID NO: 48 | rc C to T (bis1) |
| SEQ ID NO: 19 | G to A (bis2 rc) | SEQ ID NO: 49 | G to A (bis2 rc) |
| SEQ ID NO: 20 | G to A (bis2 rc) rc | SEQ ID NO: 50 | G to A (bis2 rc) rc |
| | | | |
| ANKRD13B assay 17: 29613551-29613643 | | FOXF2 assay 6:1390366-1390441 | |
| SEQ ID NO: 21 | genomic reference | SEQ ID NO: 51 | genomic reference |
| SEQ ID NO: 22 | C to T (bis1) | SEQ ID NO: 52 | C to T (bis1) |
| SEQ ID NO: 23 | rc C to T (bis1) | SEQ ID NO: 53 | rc C to T (bis1) |
| SEQ ID NO: 24 | G to A (bis2 rc) | SEQ ID NO: 54 | G to A (bis2 rc) |
| SEQ ID NO: 25 | G to A (bis2 rc) rc | SEQ ID NO: 55 | G to A (bis2 rc) rc |
| | | | |
| FOXF2 gene associated region 6:1384834-1400597 | | SEQ ID NO: 56 | ANKRD13B-F4 primer |
| | | SEQ ID NO: 57 | ANKRD13B-R4 primer |
| SEQ ID NO: 26 | genomic reference | SEQ ID NO: 58 | ANKRD13B-B7 blocker |
| SEQ ID NO: 27 | C to T (bis1) | SEQ ID NO: 59 | ANKRD13B-P4 probe |
| SEQ ID NO: 28 | rc C to T (bis1) | SEQ ID NO: 60 | FOXF2-F4 primer |
| SEQ ID NO: 29 | G to A (bis2 rc) | SEQ ID NO: 61 | FOXF2-R5 primer |
| SEQ ID NO: 30 | G to A (bis2 rc) rc | SEQ ID NO: 62 | FOXF2-B5 blocker |
| | | SEQ ID NO: 63 | FOXF2-P4 probe |

The invention is described by way of the following examples which are to be construed as merely illustrative and not limitative of the scope of the invention.

### EXAMPLE 1

### Sample preparation:

Technical samples used for assessing the assay were prepared as mixtures of bisulfite treated PBL (peripheral blood) DNA known to be unmethylated in the assay region (Human Genomic DNA: Male, Promega GmbH) and methylated DNA (DNA treated with DNA-Methyltransferases - Universal Methylated DNA, Merck Chemicals GmbH) in 10 ng/ml total. As a second technical reference control for unmethylated DNA genome wide randomly amplified DNA (Phi) was used. DNA isolated from different cancer tissues was purchased commercially from Biocat GmbH. Control DNAs and tissue DNA were bisulfite treated with the EpiTect Bisulfite Kit (Qiagen GmbH) following the manufacturers protocol.

Blood plasma samples from colorectal cancer patients and healthy individuals were collected as defined in the instructions for use (IFU) of the Epi proColon 2.0 kit (Epigenomics AG). Briefly, EDTA plasma was prepared by two centrifugation steps. Bulk plasma (a pool of plasma samples from healthy individuals; Plasma, Normal EDTA, Cliniqa Co.) was centrifuged under the same conditions before usage. Until processing, plasma samples were stored at -70°C.

### Sample/DNA processing:

Technical samples/DNA mixtures were used directly. Plasma samples were processed analogous to the pre-analytic workflow as defined in the instructions for use (IFU) of the Epi proColon 2.0 kit (Epigenomics AG).

PCR reactions either used 10 ng total DNA in each reaction for technical samples or the processed equivalent of about 1 ml plasma for plasma samples. Except for the PCR mix containing the relevant oligomers as layed out for the assays in this application, the real-time PCR on a LightCycler 480 (Roche Applied Sciences) was done following the PCR protocols of the Epi proColon 2.0 PCR kit (Epigenomics AG).

Beside methylation status of the investigated markers ANKRD13B and FOXF2, level of methylated and unmethylated Septin9 were measured to determine the total DNA yield in the individual PCR reaction. Calculation of DNA amount was done using technical DNA as a calibrator (e.g. 50% methylated DNA: 5 ng unmethylated bisulfite PBL DNA plus 5 ng methylated DNA). Methylation degree was determined by the ratio of the DNA amount of the investigated markers to the total amount of DNA (sum of methylated and unmethylated Septin9 marker).

### Results:

Methylation of the investigated markers ANKRD13B and FOXF2 could be successfully detected using the heavy methyl PCR assays described herein (Figure 2). Technical samples with only 50 pg methylated DNA (in a total background of 10 ng unmethylated DNA) show a strong signal in real time PCR. In contrast, samples processed from bulk plasma (pool of healthy individuals) show no or very faint signal in the PCR (Figure 6 and Figure 7).

The methylation status of the investigated marker was next tested on DNA derived from different cancer tissue. As shown in Figure 3B, the methylation degree of ANKRD13B in DNA from stomach, liver, colon, ovary, esophagus, bladder and prostate cancer was more than 10% in contrast to normal colon tissue (only 0.5% methylated DNA). It is expected that this strong difference of methylation in tissue can also be measured in liquid biopsies. As proof-of-concept, plasma samples from healthy individuals and CRC patiens were examined. Although mean methylation degree was only 8% in CRC plasma, samples can be clearly distinguished from healthy individuals (no detection at all - 0% methylation in plasma from healthy donors). One example of a real time PCR amplification plot detecting methylated ANKRD13B in a plasma sample of an individual CRC patient is shown in Figure 8. Beside the detection of methylated ANKRD13, signals are observed for methylated Septin 9 as a known CRC marker and unmethylated Septin 9 for the detection of unmethylated background DNA.

Figure 4B shows an amount of more than 10 % methylated DNA for FOXF2 in lung, colon, breast, stomach and esophagus cancer. Normal colon tissue shows low signals for methylated FOXF2 (only 0.2% methylated DNA). Again, as proof-of-concept for the detection of methylation in liquid biopsies, plasma samples from healthy individuals and CRC patients were examined (Figure 4A). The mean amount of methylated DNA was 10% in CRC plasma and can clearly distinguished from healthy donors (detection in merely one single sample with only 0.03% methylated target DNA). One example of a real time PCR amplification plot detecting methylated FOXF2 in a plasma sample of an individual CRC patient is shown in Figure 9. Beside the detection of methylated ANKRD13, signals are observed for methylated Septin 9 as a known CRC marker and unmethylated Septin 9 for the detection of unmethylated background DNA.

The methylation degree of both investigated markers for the individual cancer tissues are compared in a matrix shown in Figure 5. In that way a direct comparison of the methylation degree in the same kind of cancer tissue by different markers becomes obvious. Different cluster can be observed a) cancer tissues than are not methylated for the investigated marker, e.g. pancreas or kidney cancer tissue, b) cancer tissues that only show a significant amount of methylation for one marker, e.g. methylated ANKRD13 in prostate cancer tissue or methylated FOXF2 in lung cancer tissue and c) cancer tissues that are detected by both investigated markers, e.g. colon or esophagus cancer tisse. If in a measurement a cluster refered to b) is observed, specific diagnosis can be given. For the other cluster (a) and (c) further marker or a conventional diagnosis needs to be added to allow to distinguish the different forms of cancers.

## Claims

1. A method for detecting DNA methylation within genomic DNA from a sample comprising cell-free DNA from blood or a sample derived therefrom of a subject, wherein the genomic DNA has a sequence comprised in SEQ ID NO: 21 and comprises at least 4 CpG dinucleotides, and wherein the subject has an increased risk for or is suspected of having a cancer selected from the group consisting of stomach, liver, colon, ovary, esophagus, bladder and prostate cancer.

2. The method of claim 1, wherein the genomic DNA comprises at least 5 or preferably at least 6 CpG dinucleotides.

3. The method of claim 1 or 2, wherein DNA methylation is detected in a target DNA that is methylated in the cancer.

4. The method of any one of claims 1 to 3, comprising the steps of
(a) converting cytosine unmethylated in the 5-position to uracil or another base that does not hybridize to guanine in the genomic DNA;
(b) amplifying methylation-specifically a region of the converted DNA;
(c) detecting the presence or absence of DNA amplified in step (b);
wherein the presence or absence of amplified DNA is indicative of the presence or absence, respectively, of methylated genomic DNA.

5. The method of claim 4, wherein the region of the converted DNA is amplified methylation-specifically by using at least one methylation-specific oligonucleotide which is substantially identical or complementary to a stretch of contiguous nucleotides of SEQ ID NOs 22-25.

6. A method for detecting the presence or absence of cancer or a risk thereof in a subject, comprising detecting DNA methylation within genomic DNA from a sample comprising cell-free DNA from blood or a sample derived therefrom of a subject, wherein the genomic DNA has a sequence comprised in SEQ ID NO: 21 and comprises at least 4 CpG dinucleotides, and wherein the cancer is selected from the group consisting of stomach, liver, colon, ovary, esophagus, bladder and prostate cancer.

7. The method of claim 6, wherein the genomic DNA comprises at least 5 or preferably at least 6 CpG dinucleotides.

8. The method of claim 6 or 7, wherein DNA methylation is detected in a target DNA that is methylated in the cancer.

9. The method of claim 7 or 8, wherein the amount of methylated genomic DNA is determined by real-time PCR or by sequencing.

10. Use of an oligonucleotide selected from the group consisting of a primer, blocker or probe, which has a sequence that is substantially identical or complementary to a stretch of contiguous nucleotides of SEQ ID NOs 22-25, in a method for detecting DNA methylation for the diagnosis of cancer, for the assessment of the response to treatment of cancer, for the monitoring of cancer, or for the screening of subjects to detect an increased likelihood of cancer, wherein the method comprises detecting DNA methylation according to any one of claims 1 to 5, and wherein the cancer is selected from the group consisting of stomach, liver, colon, ovary, esophagus, bladder and prostate cancer.

11. Use of a kit comprising at least a first and a second oligonucleotide, wherein the first oligonucleotide and the second oligonucleotide are oligonucleotides as defined in claim 10, in a method for detecting DNA methylation for the diagnosis of cancer, for the assessment of the response to treatment of cancer, for the monitoring of cancer, or for the screening of subjects to detect an increased likelihood of cancer, wherein the method comprises detecting DNA methylation according to any one of claims 1 to 5, and wherein the cancer is selected from the group consisting of stomach, liver, colon, ovary, esophagus, bladder and prostate cancer.

12. The use of claim 11, wherein the first oligonucleotide is a probe as defined in claim 10 that is methylation-specific and the second oligonucleotide is a blocker as defined in claim 10 that is methylation-specific or a primer as defined in claim 10 that is methylation-specific.

13. The use of claim 11, wherein the first oligonucleotide is a primer as defined in claim 10 and the second oligonucleotide is also a primer as defined in claim 10 or a blocker as defined in claim 10 that is methylation-specific.

14. Use of the method of any one of claims 1-5 or of any one of claims 6-9 for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer.

## Patentansprüche

1. Verfahren zum Detektieren von DNA-Methylierung innerhalb genomischer DNA aus einer Probe, die zellfreie DNA aus Blut umfasst, oder einer davon abgeleiteten Probe von einem Subjekt, wobei die genomische DNA eine Sequenz aufweist, die in SEQ ID Nr: 21 umfasst ist und mindestens 4 CpG Dinukleotide umfasst, und wobei das Subjekt ein erhöhtes Risiko für Krebs aufweist oder unter Verdacht steht, einen Krebs aufzuweisen, ausgewählt aus der Gruppe, bestehend aus Magen-, Leber-, Darm-, Eierstock-, Speiseröhren-, Blasen- und Prostatakrebs.

2. Verfahren nach Anspruch 1, wobei die genomische DNA mindestens 5 oder vorzugsweise mindestens 6 CpG Dinukleotide umfasst.

3. Verfahren nach Anspruch 1 oder 2, wobei DNA-Methylierung in einer Ziel-DNA detektiert wird, die in dem Krebs methyliert ist.

4. Verfahren nach einem der Ansprüche 1 bis 3, umfassend die Schritte
(a) Umwandeln von unmethyliertem Cytosin in der 5-Position zu Uracil oder einer anderen Base, die nicht mit Guanin in der genomischen DNA hybridisiert;
(b) Vermehren eines Bereichs der umgewandelten DNA methylierungsspezifisch;
(c) Detektieren des Vorhandenseins oder Fehlens von in Schritt (b) vermehrter DNA;
wobei das Vorhandensein oder Fehlen vermehrter DNA das Vorhandensein beziehungsweise Fehlen methylierter genomischer DNA angibt.

5. Verfahren nach Anspruch 4, wobei der Bereich der umgewandelten DNA unter Verwendung mindestens eines methylierungsspezifischen Oligonukleotids methylierungsspezifisch vermehrt wird, das im Wesentlichen identisch oder komplementär zu einem Stück fortlaufender Nukleotide von SEQ ID Nrn. 22-25 ist.

6. Verfahren zum Detektieren des Vorhandenseins oder Fehlens von Krebs oder einem Risiko dafür bei einem Subjekt, umfassend Detektieren von DNA-Methylierung innerhalb genomischer DNA aus einer Probe, die zellfreie DNA aus Blut umfasst, oder einer davon abgeleiteten Probe von einem Subjekt, wobei die genomische DNA eine Sequenz aufweist, die in SEQ ID Nr: 21 umfasst ist und mindestens 4 CpG Dinukleotide umfasst, und wobei der Krebs aus der Gruppe ausgewählt ist, bestehend aus Magen-, Leber-, Darm-, Eierstock-, Speiseröhren-, Blasen- und Prostatakrebs.

7. Verfahren nach Anspruch 6, wobei die genomische DNA mindestens 5 oder vorzugsweise mindestens 6 CpG Dinukleotide umfasst.

8. Verfahren nach Anspruch 6 oder 7, wobei DNA-Methylierung in einer Ziel-DNA detektiert wird, die in dem Krebs methyliert ist.

9. Verfahren nach Anspruch 7 oder 8, wobei die Menge methylierter genomischer DNA durch Echtzeit-PCR oder durch Sequenzierung bestimmt wird.

10. Verwendung eines Oligonukleotids, ausgewählt aus der Gruppe, bestehend aus einem Primer, Blocker oder einer Sonde, der/die eine Sequenz aufweist, die im Wesentlichen identisch mit oder komplementär zu einem Stück fortlaufender Nukleotide von SEQ ID Nrn. 22-25 ist, in einem Verfahren zum Detektieren von DNA-Methylierung zur Diagnose von Krebs, für die Beurteilung des Ansprechens auf Krebsbehandlung, für die Überwachung von Krebs oder für die Untersuchung von Subjekten, um eine erhöhte Wahrscheinlichkeit für Krebs zu detektieren, wobei das Verfahren Detektieren von DNA-Methylierung nach einem der Ansprüche 1 bis 5 umfasst und wobei der Krebs aus der Gruppe ausgewählt ist, bestehend aus Magen-, Leber-, Darm-, Eierstock-, Speiseröhren-, Blasen- und Prostatakrebs.

11. Verwendung eines Kits, das mindestens ein erstes und ein zweites Oligonukleotid umfasst, wobei das erste Oligonukleotid und das zweite Oligonukleotid in Anspruch 10 definierte Oligonukleotide sind, in einem Verfahren zum Detektieren von DNA-Methylierung zur Diagnose von Krebs, für die Beurteilung des Ansprechens auf Krebsbehandlung, für die Überwachung von Krebs oder für die Untersuchung von Subjekten, um eine erhöhte Wahrscheinlichkeit für Krebs zu detektieren, wobei das Verfahren Detektieren von DNA-Methylierung nach einem der Ansprüche 1 bis 5 umfasst und wobei der Krebs aus der Gruppe ausgewählt ist, bestehend aus Magen-, Leber-, Darm-, Eierstock-, Speiseröhren-, Blasen- und Prostatakrebs.

12. Verwendung nach Anspruch 11, wobei das erste Oligonukleotid eine in Anspruch 10 definierte Sonde ist, die methylierungsspezifisch ist, und das zweite Oligonukleotid ein in Anspruch 10 definierter Blocker, der methylierungsspezifisch ist, oder ein in Anspruch 10 definierter Primer ist, der methylierungsspezifisch ist.

13. Verwendung nach Anspruch 11, wobei das erste Oligonukleotid ein in Anspruch 10 definierter Primer ist und das zweite Oligonukleotid auch ein in Anspruch 10 definierter Primer oder ein in Anspruch 10 definierter Blocker ist, der methylierungsspezifisch ist.

14. Verwendung des Verfahrens nach einem der Ansprüche 1-5 oder nach einem der Ansprüche 6-9 für die Diagnose von Krebs, für die Beurteilung des Ansprechens auf Krebsbehandlung, für die Überwachung von Krebs oder für die Untersuchung von Subjekten, um eine erhöhte Wahrscheinlichkeit für Krebs zu detektieren, wobei der Krebs aus der Gruppe ausgewählt ist, bestehend aus Magen-, Leber-, Darm-, Eierstock-, Speiseröhren-, Blasen- und Prostatakrebs.

## Revendications

1. Procédé de détection de méthylation d'ADN avec un ADN génomique à partir d'un échantillon comprenant de l'ADN acellulaire issu de sang ou d'un échantillon dérivé de celui-ci d'un sujet, dans lequel l'ADN génomique présente une séquence comprise dans SEQ ID NO : 21 et comprend au moins 4 dinucléotides CpG, et dans lequel le sujet présente un risque augmenté de cancer ou est suspecté d'avoir un cancer sélectionné à partir du groupe consistant en cancer de l'estomac, du foie, du côlon, de l'ovaire, de l'œsophage, de la vessie et la prostate.

2. Procédé selon la revendication 1, dans lequel l'ADN génomique comprend au moins 5 ou de préférence au moins 6 dinucléotides CpG.

3. Procédé selon la revendication 1 ou 2, dans lequel une méthylation d'ADN est détectée dans un ADN cible qui est méthylé dans le cancer.

4. Procédé selon l'une quelconque des revendications 1 à 3, comprenant les étapes consistant à
(a) convertir de la cytosine non méthylée dans la position 5 en uracile ou une autre base qui ne s'hybride pas en guanine dans l'ADN génomique ;
(b) amplifier spécifiquement en méthylation une région de l'ADN converti ;
(c) détecter la présence ou l'absence d'ADN amplifié à l'étape (b) ;
dans lequel la présence ou l'absence d'ADN amplifié est indicative de la présence ou de l'absence, respectivement, d'ADN génomique méthylé.

5. Procédé selon la revendication 4, dans lequel la région de l'ADN converti est amplifiée spécifiquement en méthylation en utilisant au moins un oligonucléotide spécifique à la méthylation qui est sensiblement identique ou complémentaire à un étirement de nucléotides contigus de SEQ ID NO : 22-25.

6. Procédé de détection de la présence ou de l'absence de cancer ou d'un risque de celui-ci chez un sujet, comprenant la détection d'une méthylation d'ADN dans un ADN génomique à partir d'un échantillon comprenant de l'ADN acellulaire issu de sang ou d'un échantillon dérivé de celui-ci d'un sujet, dans lequel l'ADN génomique présente une séquence comprise dans SEQ ID NO : 21 et comprend au moins 4 dinucléotides CpG, et dans lequel le cancer est sélectionné à partir du groupe consistant en cancer de l'estomac, du foie, du côlon, de l'ovaire, de l'œsophage, de la vessie et la prostate.

7. Procédé selon la revendication 6, dans lequel l'ADN génomique comprend au moins 5 ou de préférence au moins 6 dinucléotides CpG.

8. Procédé selon la revendication 6 ou 7, dans lequel une méthylation d'ADN est détectée dans un ADN cible qui est méthylé dans le cancer.

9. Procédé selon la revendication 7 ou 8, dans lequel la quantité d'ADN génomique méthylé est déterminée par PRC en temps réel ou par séquençage.

10. Utilisation d'un oligonucléotide sélectionné à partir du groupe consistant en une amorce, un bloqueur ou une sonde, qui présente une séquence qui est sensiblement identique ou complémentaire à un étirement de nucléotides contigus de SEQ ID NO : 22-25, dans un procédé de détection d'une méthylation d'ADN pour le diagnostic de cancer, pour l'évaluation de la réponse à un traitement du cancer, pour la surveillance du cancer, ou pour le criblage de sujets afin de détecter une probabilité augmentée de cancer, dans laquelle le procédé comprend la détection d'une méthylation d'ADN selon l'une quelconque des revendications 1 à 5, et dans laquelle le cancer est sélectionné à partir du groupe consistant en cancer de l'estomac, du foie, du côlon, de l'ovaire, de l'œsophage, de la vessie et la prostate.

11. Utilisation d'un kit comprenant au moins un premier et un second oligonucléotide, dans laquelle le premier oligonucléotide et le second oligonucléotide sont des oligonucléotides tels que définis dans la revendication 10, dans un procédé de détection d'une méthylation d'ADN pour le diagnostic de cancer, pour l'évaluation de la réponse à un traitement du cancer, pour la surveillance du cancer, ou pour le criblage de sujets afin de détecter une probabilité augmentée de cancer, dans laquelle le procédé comprend la détection d'une méthylation d'ADN selon l'une quelconque des revendications 1 à 5, et dans laquelle le cancer est sélectionné à partir du groupe consistant en cancer de l'estomac, du foie, du côlon, de l'ovaire, de l'œsophage, de la vessie et la prostate.

12. Utilisation selon la revendication 11, dans laquelle le premier oligonucléotide est une sonde telle que définie dans la revendication 10 qui est spécifique à la méthylation et le second oligonucléotide est un bloqueur tel que définie dans la revendication 10 qui est spécifique à la méthylation ou une amorce telle que définie dans la revendication 10 qui est spécifique à la méthylation.

13. Utilisation selon la revendication 11, dans laquelle le premier oligonucléotide est une amorce telle que définie dans la revendication 10 et le second oligonucléotide est également une amorce telle que définie dans la revendication 10 ou un bloqueur tel que défini dans la revendication 10 qui est spécifique à la méthylation.

14. Utilisation du procédé selon l'une quelconque des revendications 1 à 5 ou selon l'une quelconque des revendications 6 à 9 pour le diagnostic du cancer, pour l'évaluation de la réponse à un traitement du cancer, pour la surveillance du cancer, ou pour le criblage de sujets afin de détecter une probabilité augmentée de cancer, dans laquelle le cancer est sélectionné à partir du groupe consistant en cancer de l'estomac, du foie, du côlon, de l'ovaire, de l'œsophage, de la vessie et la prostate.
